# EUROPEAN PATENT APPLICATION

(11) **EP 4 613 461 A1**
(43) Date of publication of application: **10.09.2025**
(21) Application number: 25161761.9
(22) Date of filing: 05.03.2025
(51) Int. Cl.: B29C 64/386, B22F 10/80, B33Y 50/00, B22F 10/38, B22F 10/366, B22F 10/18, B29C 64/118, B33Y 80/00

(54) **THREE DIMENSIONAL (3D) PRINTING SYSTEM AND METHOD, ALSO PRINTED OBJECT.**

(30) Priority: 05.03.2024 NL 2037180
(71) Applicant: DEMCON Bond 3D B.V., 7521 PK Enschede (NL)
(72) Inventor: Wimmenhove, Tom, 7521 PK Enschede (NL); Kuiper, Guus, 7521 PK Enschede (NL)
(74) Representative: Arnold & Siedsma

(57) **Abstract**

The disclosure relates to a method for three dimensional (3D) printing an object, the method comprising the steps of: - providing a print job, the print job comprising a representation of the object, - dividing the representation of the object in one or more bodies;- for each body, define settings for printing the respective body, wherein settings for at least one body include filling the body with a porous infill structure; - slicing the representation of the object in a number of slices, - the step of slicing comprising, for the body to be filled with the porous infill structure: - defining for every slice an infill area (64) that is to be filled with the porous infill structure; - defining a circumference (68) of the infill area; - adapting the porous infill structure to the circumference; and - filling the infill area with the adapted infill structure.

## Description

### FIELD OF THE INVENTION

The invention relates to manufacturing a three-dimensional (3D) object by additive manufacturing. Additive manufacturing may also be referred to as 3D printing. The 3D printing method may include extrusion based modelling. Herein, a number of slices, each slice comprising tracks of modelling material, are deposited on top of each other by means of a printhead connected to a three dimensional positioning system.

### BACKGROUND

In three-dimensional modelling, objects are formed by depositing multiple subsequent layers of modelling material in a controlled manner to create a desired three-dimensional (3D) object. This way of forming objects can be referred to as additive manufacturing or three dimensional (3D) printing. For three-dimensional modelling a three-dimensional printer may be used. The printer may have one or more printheads for dispensing the modelling material on a base, while the printhead and base are moved with respect to each other.

The object to be printed can be placed on a build plate. The build plate or build surface may be arranged on a base. The build plate may be removable. In some printers, the base is the frame of the printer. The printhead may be movable in one or more dimensions relative to the object and/or vice versa the base including the object may be movable in one or more dimensions relative to the printhead. Various combinations are possible for moving the base relative to the printhead and vice versa.

Motion of the printhead with respect to the base are controlled by a control system. The control system controls a positioning system to which the printhead and/or build plate is attached. The positioning system may be able to move one or both of the printhead or build plate in one or more directions. The positioning system may for instance be able to provide translations in three dimensions, or to also provide rotations up to a total of six degrees of freedom. By means of software a pattern of tracks can be designed, which is used for moving the printhead and build plate relative to each other and for depositing the tracks.

For instance patent document US2020070405 describes details of a 3D printer and a method to use a vacuum clamped platen on an air bearing for 3D printing objects.

The object is created on the base structure. The modelling material can be fused with previously formed tracks. The modelling material can be supplied to the printhead in the form of, for example, filaments, granulate, rods, liquid or a suspension.

The printhead may dispense the modelling material from the printhead through a nozzle and deposit it on the base in the form of tracks forming a layer of tracks. When a previous layer of the object has been deposited, the subsequent layer is deposited on the previous layer. The deposited material is allowed to solidify. The modelling material can be thermally or chemically or otherwise fused with the previously deposited tracks. The modelling material can be dispensed from the printhead and deposited on the build plate and/or on or next to previously deposited tracks to solidify after deposition.

The relative motion of the base and the printhead and simultaneous deposition of modelling material from the printhead allows the object to grow with each deposited layer to gradually attain its desired shape. Infill structures are normally deployed on the insides of parts, or bodies thereof, to reduce the printing time and material usage. Infill structures can also be used on the outside of parts to create open structures, for instance to create a fluid filter or to make a porous structure on a medical implant that promotes bone ingrowth. Typically, in infill methods according to the prior art, a unit cell of an infill structure is repeated in an orthogonal grid in a (global or local) coordinate system. For instance, Prusa Research [Prague, Czech Republic] offers slicer software, named PrusaSlicer. According to the website of Prusa Research, "PrusaSlicer is an open-source, feature-rich, frequently updated tool that contains everything you need to export the perfect print files for your 3D printer." The PrusaSlicer typically uses orthogonal grids for infill structures.

When such infill structure is deployed in a volume with a curved surface, the orthogonal repetition of the unit cells causes a problem. For instance, loose ends of the clipped units may result in loose end pieces on the outside of the structure. These loose ends are typically unsupported by an underlying structure and therefore not printed accurately. Also, the ends are rough and may deform when the structure is exposed to a load. When porous structures are intended to be used as medical implants, surface smoothness is of particular importance. If the structures are not sufficiently smooth, they may cause harm to tissue during insertion, or the forces of insertion may damage the loose ends, potentially harming the patient. Also, these loose ends are aesthetically disagreeable.

US11135771 and US11481886 disclose methods for 3D printing a medical implant. In some embodiments, respective porous regions of an object may have different porosity. A pattern may be wavy or sinusoidal. In some embodiments, upon reaching a perimeter, a print head can be moved in the x-y plane and continue depositing printing material in a wave pattern back in the direction towards the interior of a medical implant until reaching the perimeter again. In some embodiments, the printing pattern or porosity may be altered between each slice, providing for multiple printing patterns and porosities within the fully formed medical implant. For example, in some embodiments, a second layer is deposited on top of a first layer in a wave pattern in the same design as present in a first layer. However, the pattern can be rotated at a predetermined angle or degree, whereby printing material is not deposited in the exact same layout, and instead, there is a crisscrossing effect of printing material between the first layer and the second layer.

The structures disclosed in US11135771 and US11481886 are variants to the line infill. It poses disadvantages similar to other known infills for porous structures, due to connected lines at the perimeter of an object and the associated drop in porosity.

The present disclosure aims to provide an alternative 3D printing system and method, overcoming at least some of the disadvantages described above.

### SUMMARY OF THE INVENTION

According to one aspect of the invention, a method for three dimensional (3D) printing an object is provided, the method comprising the steps of:
- providing a print job, the print job comprising a representation of the object,
- dividing the representation of the object in one or more bodies;
- for each body, define settings for printing the respective body, wherein settings for at least one body include filling the body with a porous infill structure;
- slicing the representation of the object in a number of slices,
- the step of slicing comprising, for the body to be filled with the porous infill structure:

- defining for every slice an infill area that is to be filled with the porous infill structure;
- defining a circumference of the infill area;
- adapting the porous infill structure to the circumference; and
- filling the infill area with the adapted infill structure.

In an embodiment, the step of adapting the porous infill structure to the circumference comprises one or more of:
- changing a pattern at least in part of non-orthogonal repetition;
- executing a non-linear coordinate transformation of the infill structure;
- adding additional tracks in parts of the infill pattern with low density.

In an embodiment, the step of adapting the porous infill structure to the circumference comprises changing the infill pattern to at least in part follow a curvature of the circumference.

In an embodiment, the method comprises the step of printing the sliced print job to provide the object.

In an embodiment, the porous infill structure comprises a pattern having a unit cell which is repeated in an orthogonal grid.

In an embodiment, the method comprises the step of adapting the porous infill structure to the circumference including rendering at least part of the infill structure non-orthogonal.

In an embodiment, the step of adapting the porous infill structure to the circumference includes changing a repetitive pattern of at least part of the infill structure.

In an embodiment, the step of slicing comprises, for the body to be filled with the porous infill structure:
- identify one or more points on the circumference of the infill area;
- connecting the one or more points with one or more extended lines;
- creating offsets to the one or more extended lines towards an inside of the respective slice;
- trimming the offsets to the circumference of the infill area.

In an embodiment, the step of slicing comprises, for the part to be filled with the porous infill structure:
- filling the infill area with the porous infill structure;
- for a percentage of the slices of the respective part, adding tracks following the circumference of the infill area; and
- removing tracks of the infill structure which are within a threshold distance with respect to the circumference of the infill area.

In an embodiment, the step of slicing the part to be filled with the infill structure comprises:
- identify one or more points on the circumference of the infill area;
- connecting the one or more points with one or more lines, said lines defining the circumference.

In an embodiment, the percentage is in the range of 25 to 75%, for instance about 50%.

In an embodiment, the step of slicing comprises, for the part to be filled with the infill structure:
- adapting a grid to the circumference of the infill area to create a virtual grid;
- projecting the virtual grid on the infill area;
- projecting the infill structure on the virtual grid.

In an embodiment, the step of adapting a grid to the circumference of the infill area to create a virtual grid comprises changing a distance between tracks of the grid within a range.

In an embodiment, the step of adapting a grid to the circumference of the infill area to create a virtual grid comprises changing a distance between adjacent ends of tracks of the grid within a range.

In an embodiment, the step of adapting a grid to the circumference of the infill area to create a virtual grid comprises changing a number of tracks of the grid within a range to optimize an average distance between said tracks.

According to another aspect, the disclosure provides a 3D printing system adapted to execute a method as described above.

According to another aspect, the disclosure provides a three-dimensional object made using the method as described above.

According to another aspect, the disclosure provides a three-dimensional printed object, comprising:
at least two bodies, wherein at least one body has a porous structure extending to an outer surface of the object;
wherein a majority of ends of first tracks of the porous structure facing the outer surface are supported by second tracks.

In an embodiment, an unsupported length m of the ends of the first tracks facing the outer surface is at most 1.99, 1.8, 1.69, 1.5, 1.4, 1.39, 1.2, 1.19, 1, 0.99, 0.9, 0.84, 0.8, 0.7, 0.69, 0.65, 0.6, 0.59, 0.55, 0.5, 0.49 times a track width of said first tracks.

In an embodiment, the ends of the first tracks facing the outer surface have a radius R1 exceeding 0.35 times the track width of the first tracks.

In an embodiment, the radius R1 is in the order of 0.4, 0.42, 0.44, 0.45, 0.46, 0.50, 0.53, 0.55, 0.57, 0.6., 0.64, 0.65, 0.68, 0.7 times the track width of the first tracks or more.

In an embodiment, the radius R1 is in the range of about 0.3 to 0.85 times the track width of the first tracks.

In an embodiment, at least 50%, 51%, 55%, 60%, 61%, 63%, 65%, 70%, 71%, 75%, 80%, 81%, 91% of the ends of the first tracks of the porous structure facing the outer surface are supported by second tracks.

In an embodiment, spacings (ph) in between respective tracks provide openings to the internal volume of the object, thereby providing porosity, wherein the spacings are at least 0,1 times the width of the tracks.

In an embodiment, the openings are at least 0.2, for instance about 0.3 times the width of the tracks.

In an embodiment, the porosity exceeds 40%. Porosity herein may refer to surface porosity. The porosity may exceed 43%, for instance about 45%, 48%, 50%, 55%, 60%, 65%, 70%, or 75%.

In an embodiment, a track width is about 1.2 to 10 times a layer height.

In an embodiment, the object is a medical implantable device or a filter. The medical implantable device may be selected from:
- Craniomaxillofacial implant;
- Trauma plates;
- Cervical implants;
- Lumbar interbody fusion implants (spine, thorax region and lower back);
- Hip cups;
- Knee implants; and
- Ankle plates.

In an embodiment, the object is made of a material comprising a thermoplastic polymer composition selected from the group of polyethylene, polypropylene, ABS, polycarbonate, polyamide and polyarylether ketones (PAEK), polyether ketone (PEK), polyether ether ketone (PEEK), polyether ketone ketone (PEKK), polyether ether ketone ketone (PEEKK), polyether ether ketone ether ketone ketone (PEKEKK), and combinations thereof.

In an embodiment, the thermoplastic polymer composition comprises at least 80 wt% of a PAEK, for instance at least 90 wt% of a PAEK, or the thermoplastic polymer composition comprises at least 80 wt% of a PEEK, for instance at least 90 wt% of a PEEK, for instance 100 wt% PEEK.

### BRIEF DESCRIPTION OF THE DRAWINGS

Figure 1 schematically shows an example of a 3D printing system;
Figure 2 shows a perspective view of a digital model of an exemplary object to be 3D printed, wherein the model includes two bodies each having different settings for printing;
Figure 3 shows a perspective view of a partly printed object based on the model of Fig. 2 during printing thereof, including one body having a conventional porous infill structure;
Figure 4 shows a top view of a cross section of the model of Fig. 2, wherein one body comprises a first pattern of a conventional porous infill structure;
Figure 5 shows a top view of a cross section of the model of Fig. 2, wherein one body comprises a second pattern of a conventional porous infill structure;
Figure 6 shows a perspective view of an exemplary curved structure;
Figure 7 shows an exemplary outline of a body;
Figure 8 shows the body of Fig. 7 when printed using a conventional method;
Figure 9 shows an exemplary outline of another body;
Figure 10 shows the body of Fig. 9 when printed using a conventional method;
Figure 11 shows an exemplary outline of yet another body;
Figure 12 shows the body of Fig. 11 when printed using a conventional method;
Figure 13 shows the body of Fig. 7 printed using an embodiment of a method of the disclosure;
Figure 14 shows the body of Fig. 9 printed using an embodiment of a method of the disclosure;
Figure 15 shows the body of Fig. 11 printed using an embodiment of a method of the disclosure;
Figure 16 shows a perspective view of a detail of an exemplary structure having a curved surface which has been 3D printed using an embodiment of a method of the present disclosure;
Figures 17, 18, and 19 show perspective views of the model of Fig. 2, wherein at least one body is provided with respective examples of porous infill structures printed using a method of the disclosure;
Figure 20 shows a top view of a detail of an exemplary printed pattern of tracks;
Figure 21 shows a side view of the exemplary pattern of Fig. 20;
Figures 22 to 36 exemplify subsequently printing steps and the associated printed layer according to a method of the disclosure;
Figures 37, 38 and 39 respectively show a perspective view, top view and side view of a detail of a 3D printed object;
Figures 40 to 44 show top views of subsequent steps to create a model according to an embodiment of a method of the disclosure;
Figures 45 to 50 show top views of subsequent steps to create a model according to another embodiment of a method of the disclosure;
Figures 51 to 56 show top views of subsequent steps to create a model according to yet another embodiment of a method of the disclosure;
Figures 57, 58 and 59 show top views of subsequent steps to create a model according to an embodiment of a method of the disclosure;
Figure 60A shows a perspective view of an exemplary end of a 3D printed track of a porous body, printed using a method according the present disclosure;
Figure 60B shows a perspective view of an exemplary end of a 3D printed track of a porous body, printed using a conventional printing method and subsequent etching of a non-porous surface of the body;
Figure 60C shows a perspective view of an exemplary end of a 3D printed track of a porous body, printed using a conventional printing method and subsequent etching of a non-porous surface of the body with subsequent heat treatment of the end;
Figures 61A, 61B and 61C respectively show a top view, side view and front view of the end of the track shown in Fig. 60A;
Figures 62A, 62B and 62C respectively show a top view, side view and front view of the end of the track shown in Fig. 60B; and
Figures 63A, 63B and 63C respectively show a top view, side view and front view of the end of the track shown in Fig. 60C.

### DETAILED DESCRIPTION

The following terms and phrases as used herein may be construed as follows.

"3D printing" or "additive manufacturing" (AM) is the construction of a three-dimensional object from a CAD model or a digital 3D model. It can be done in a variety of processes in which material is deposited, joined or solidified under computer control, with material being added together (such as plastics, liquids or powder grains being fused), typically layer by layer. The term additive manufacturing can be used synonymously with 3D printing. One of the key advantages of 3D printing is the ability to produce very complex shapes or geometries that would be otherwise impossible to construct by hand, including hollow parts or parts with internal truss structures to reduce weight.

Additive manufacturing (AM) processes can be divided into categories. The ASTM F42 committee released a classification of seven additive manufacturing techniques:
Binder jetting: an additive manufacturing process in which a liquid bonding agent is selectively deposited to join powder materials;
Directed energy deposition (DED): an additive manufacturing process in which focused thermal energy is used to fuse materials by melting as they are being deposited;
Material extrusion (ME): an additive manufacturing process in which material is selectively dispensed through a nozzle or orifice;
Material jetting: an additive manufacturing process in which droplets of build material are selectively deposited;
Powder bed fusion: an additive manufacturing process in which thermal energy selectively fuses regions of a powder bed;
Sheet lamination: an additive manufacturing process in which sheets of material are bonded to form an object; and
Vat photopolymerization: an additive manufacturing process in which liquid photopolymer in a vat is selectively cured by light-activated polymerization.

"Material extrusion" or "extrusion based modelling" is an additive manufacturing technique that uses a filament of material, such as thermoplastic or composite material, to construct 3D parts. The filament may be continuous, i.e. spooled, may be provided as granules, or may be in the form of rods of material. The material in the form of plastic filament is fed through an extruding nozzle, where it is heated and then deposited onto the build platform layer by layer. Material extrusion technology was first developed in the 1980s under the registered name of "fused deposition modeling" (FDM), by Stratasys Inc. "Fused filament fabrication" (FFF) is another name for a technique similar to FDM. falls under this category. Also slurries can be printed, for instance, to create ceramic parts. Here, the transition from fluid to solid is chemical.

"Build plate" as used herein refers to a plate or plate like structure suitable as a support for printing an object. The build plate may be fixed in the printer, or may be removable to allow replacement.

An "object" as used herein refers to an object to be printed. The term "part" may be used as well as object.

A "body" as used herein is a subsection of an object, having certain settings for 3D printing. An object may have multiple bodies, wherein respective bodies may be 3D printed using different settings.

A "print job" is a file or set of files comprising instructions to be printed with a printer. Jobs may typically be identified by a unique number, and may be assigned to a particular destination, such as a certain printer. Jobs can also have options associated with them such as media size, number of copies and priority. In the context of the present disclosure, a print job may typically comprise one or more objects to be printed in three dimensions by a 3D printer.

A "track" may typically relate to a printed line of extruded material.

An "infill volume" may typically relate to the volume of a body to be provided with an infill structure.

"Post processing" may generally refer to any treatment to modify a 3D printed object after printing. Treatment herein may include removing material or changing the shape of the printed material. Removing material may include, for instance, one or more of, but not limited to, machining, blasting, grinding, polishing, water jetting, laser ablation, chemical treatment, or any other method. Changing the shape may include, for instance, heat treatment, chemical vapor treatment, or any other method.

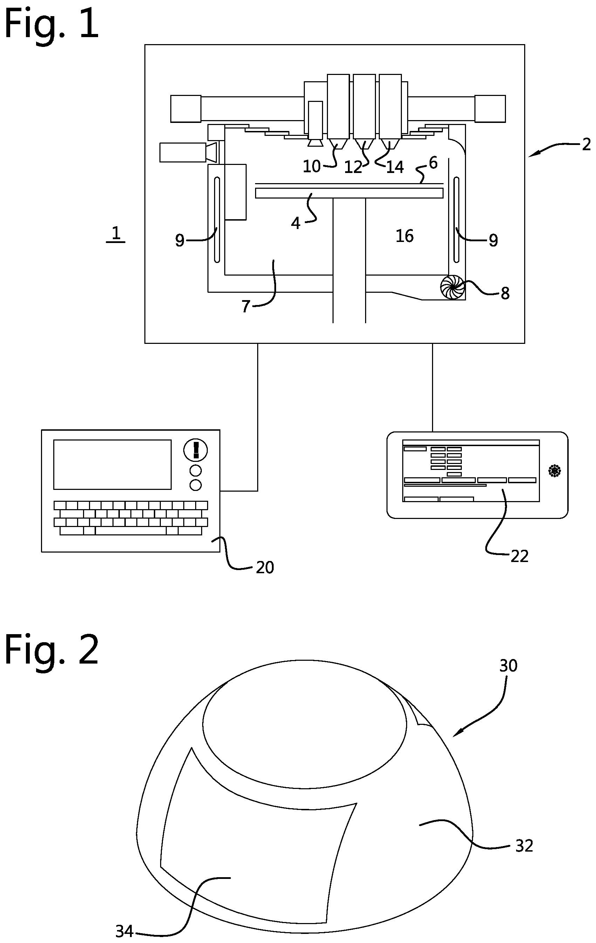

Generally referring to Figure 1, the printer or printing system may be based on Fused Filament Fabrication (FFF). The idea is to print material, wherein the material is the central focus. This means the environment has to be adaptable to the printed material. This can be done by thermally controlled print heads (multiple temperature controlled stages), pressure and flow controlled extruders (combination of feeder and print head) and a thermally stable environment. Referring to Figure 1, three extruders may be used to print parts. These extruders can be arranged side by side on a platform. The platform can be movable in the horizontal plane, referred to as motion along the x-axis and the y-axis. The extruded material is deposited on a build plate. The build plate may be heated via a temperature controlled bed. The bed may be movable, for instance at least vertically. Vertical movement may be referred to as motion along the z-axis.

It is common to use materials that have been adapted to make them easier to print, for instance, to improve melt viscosity, to inhibit crystallization, to reduce shrinkage during cooldown, or otherwise. Alternatively, shrinkage of the material after printing and upon cooling may be taken into account, for instance by compensating in the model or the toolpaths, in order to print pre-certified materials or materials preferred by customers. Herein, shrink is dealt with in other ways, for instance compensated by software.

Generally referring to Figure 1, a system 1 for three dimensional (3D) printing an object may comprise a housing 2. The housing may comprise a table 4 having a top surface for holding a build plate 6. The table may be a vacuum table, wherein the pressure differential due to the vacuum can hold or release the build plate. The vacuum table typically has a top surface provided with a plurality of openings. A vacuum source can be connected to the plurality of openings for reducing a pressure between the vacuum table and the build plate. The volume inside the housing 2 may be referred to as build room 7. The system may comprise one or more fans 8 for circulating air in the build room 7.

The system may also be provided with a heating system. The heating system may comprise one or more heating elements 9 for heating one or more of the build room 18 and/or the top surface of the table 4.

One or more extrusion heads 10, 12, 14 are typically provided for printing the object on the build plate by extruding a thermoplastic material and depositing the thermoplastic material along tracks onto the build plate to print a first layer and subsequently to print additional layers on top of a previous layer. The system typically includes a mechanism to move the print heads 10, 12, 14 and/or the table 4 including the build plate 6 with respect to each other. Said mechanism may include a lift 16 to move the table, for instance up or down. Alternatively the mechanism may be able to adjust the position of one or more of the print heads with respect to the table 4.

The system 1 may typically include a controller 20 and a display 22. The controller, such as a computer, may control the printing process and execute various tasks such as slicing and providing digitized models of the objects to be printed.

An object to be printed can be converted to a digital model. The digital model may be a computer aided design, or CAD model. CAD models can be saved in a stereolithography file format (STL), a de facto CAD file format for additive manufacturing that stores data based on triangulations of the surface of CAD models.

Once completed, the STL file needs to be processed by a piece of software called a "slicer". The slicer converts the digital model into a series of thin layers, also referred to as slices. The software produces a G-code file containing instructions tailored to a specific type of 3D printer. This G-code file can then be printed with 3D printing client software (which loads the G-code, and uses it to instruct the 3D printer during the 3D printing process). A pattern of tracks is designed by means of this dedicated software. The software determines a pattern of tracks in each slice. The pattern of tracks is used to move the printhead relative to the base and to deposit the tracks of modelling material in each layer. The terms "slice" and "layer" may be used interchangeably.

Generally referring to Figure 2, a print job may generally comprise at least one object 30. An object to be printed may also be referred to as a "part". Figure 2 indicates a model, i.e. a digital representation, of an exemplary object. An object may be comprised of one or more bodies 32, 34. Herein, a body may be defined as a section of the object having certain characteristics. For each body, dedicated slicer settings can be determined, in accordance with said characteristics. Some settings, such as build room temperature, can be set on print job level, but not for each body individually. Other slicer settings however can be set specifically for a respective body. In the example of Figure 2, the first body 32 may be printed with a relatively solid pattern. The second body 34 may have settings indicating that the respective body should be printed using a porous structure.

The operator of the slicer can typically define one or more of:
- The type of infill structure;
- The pitch of the infill structure;
- The repetition pattern.

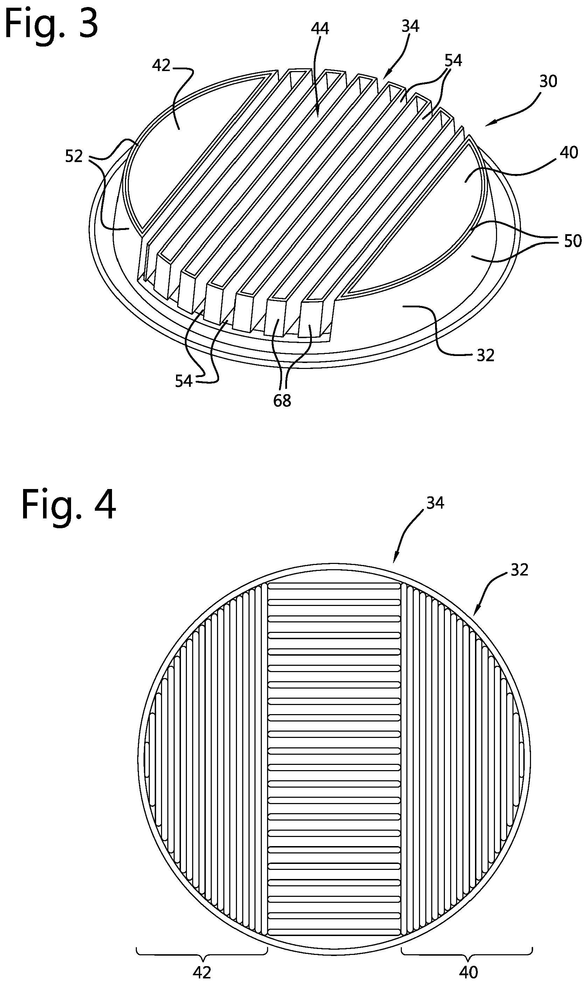

Generally referring to Figure 3, using the settings for each body, a slicer can slice the model 30 and provide a sliced print job. A sliced print job is typically divided in layers, every slice representing a layer. The 3D printer can print the print job layer after layer, printing one layer on top of a previous layer. In some of the slices, sections of a respective body may be disconnected. As exemplified in Figure 3, in some layers, the first body 32 may be divided in two islands 40, 42. In the same layer, the second body 34 may be contiguous, comprising only a single island 44. For each island, print paths for the printhead are determined based on the respective settings.

3D printed objects may have bodies printed ranging from solid (100% infill) to completely hollow. If a body is not printed solid, a method can be used that fills the inside of the respective body with an infill structure having a certain porosity. Porosity herein may be defined as the ratio of total volume of the material over total volume of the respective body. The latter may be referred to as volumetric porosity.

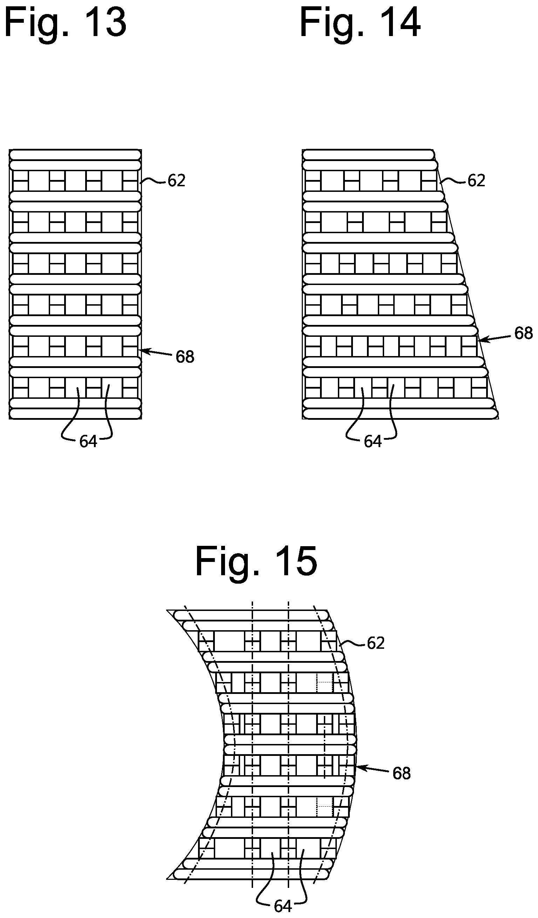

A difference between a porous body, and a solid body, is the outer surface thereof. A porous body has an outer surface having an open structure. Thus, the porous body has openings on the outside providing an opening to pores or other open structures of the infill structure inside the respective body. A solid body on the other hand typically has a closed
outer surface. In other words, infill structures are normally deployed on the insides of bodies to reduce the printing time and material usage. Infill structures can also be used on the outside of bodies to create open structures, for instance to create a fluid filter or to create a porous structure for a medical implant that promotes bone ingrowth. Porosity of the outer surface of the porous body herein may be defined as the percentage of surface area which is open with respect to the total area of the respective surface. Reference may be made to surface porosity. Examples of these structures can be seen in fig. 13 to 15.

As exemplified in Figures 3 to 5, the first body 32, with Fig. 3 showing islands 40 and 42 thereof, is printed having a closed outer surface 50, 52. The second body 34 is porous. Herein, the body 34 is printed having at least some openings 54 in the outer surface of the respective body. Figure 3 exemplifies a porous body 34 printed according to a conventional method.

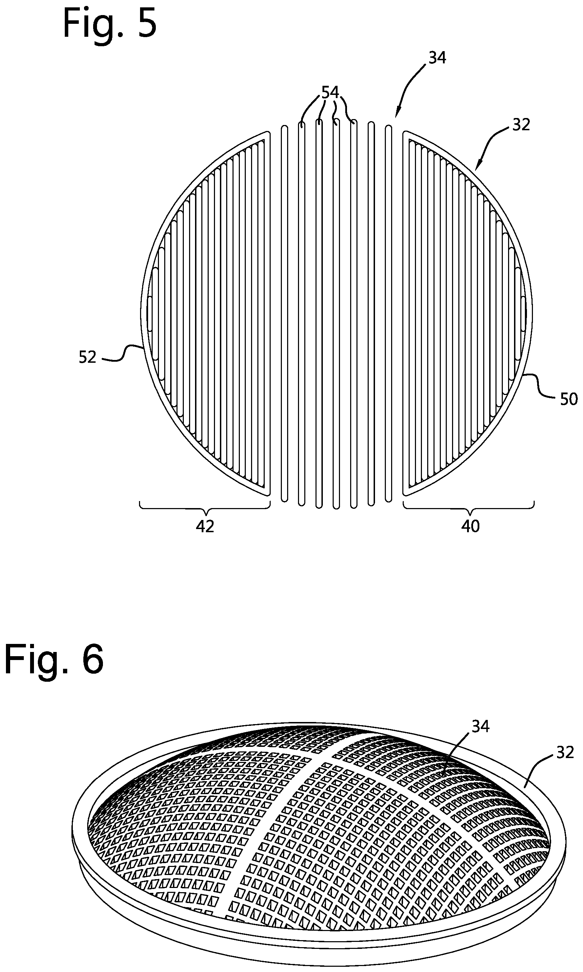

Figure 4 exemplifies tracks printed in a first layer. Figure 5 exemplifies tracks printed in a second layer. Referring to Figure 2, the object may be 3D printed by printing the track patterns shown in Figures 4 and 5 intermittently.

Figure 6 shows an example of an object having a first, non-porous body 32 and a second, porous body 34. The object shown in Fig. 6 may be used as a filter.

To distinguish with respect to an infill used to fill the internal space of a non-porous body, herein below the infill structures of a porous body may be referred to as a porous infill structure.

Infill structures as such may have many different shapes. Various types can be defined. Most infill patterns are assembled from, for instance, two or more types of structures which are repeated in a grid. Herein, each layer type can be repeated any number of times before switching to another type. For instance, a certain first pattern can be repeated a number of times (from 1 to 5 times, for instance 3 times), and then a second pattern B can be repeated a number of times (from 1 to 5 times, for instance 2 times) before switching back to the first pattern A. Infill structures include, for instance:
i) Rectilinear. Rectilinear is one of the basic infill patterns. It creates a rectilinear grid by printing one layer in one direction, and a subsequent layer rotated by 90°, etc. In top view, this infill may look like a pattern of squares.
ii) Aligned rectilinear is formed by parallel lines drawn inside the body of a model. See Figure 3 for an example. Similar to rectilinear, this infill saves time, has average material consumption, and it does not accumulate material at crossings.
iii) Grid is one of the simplest and fastest variants of infill. Unlike rectilinear, grid is printed in both directions, rotated by 90°, in each layer. This way, material accumulates in
   spots where the paths cross. The grid infill is more solid (and has better layer adhesion) than the rectilinear infill.
iv) Triangles. An infill using triangles works similarly to the grid infill. The paths cross in one layer, however, this time they are printed in three directions and form a triangle structure. Material and time consumption is almost identical to the grid.
v) Stars is an infill based on triangles but paths are shifted to make six-pointed stars. Again, this infill is created by lines that cross each other within a single layer. Material and time consumption is similar to the previous infill.
vi) Cubic is an infill with paths that cross each other within one layer. However, unlike previously described infills, it creates cubes oriented with one corner facing down. This way it makes numerous air pockets that might serve as heat insulation, or cause the object to float on water (with waterproof filaments such as PETG).
vii) Line is an infill using lines, whereas in each layer the direction of the lines is slightly rotated with respect to the lines in other layers. The lines infill does not feature any crossing paths in one layer. Its paths are similar to the rectilinear infill but they are not parallel to each other. Instead, they are printed at an acute angle. This infill is similar to rectilinear when it comes to printing time and material consumption.
viii) Concentric. The concentric infill traces the model perimeter lines and makes them smaller towards the centre of a body. For instance, if the body is a cylinder, the concentric infill will create concentric circles inside that cylinder. Material consumption is comparable to previous types of infill patterns. As the concentric infill pattern replicates the perimeter line, a respective body printed using this infill typically has a fully closed, i.e. non-porous, perimeter.

As referenced in the introduction, most conventional infill methods deploy a certain pattern having a unit cell which is repeated in an orthogonal grid, as defined in a (global or local) coordinate system. In addition to infill patterns based on repeated unit cells, as described above, it is possible to define an infill as a mathematical function of the height (z-coordinate). Such structures may include, for instance:
ix) a Hilbert curve,
x) a gyroid infill structure, or
xi) organic structures having a (substantially) random pattern.

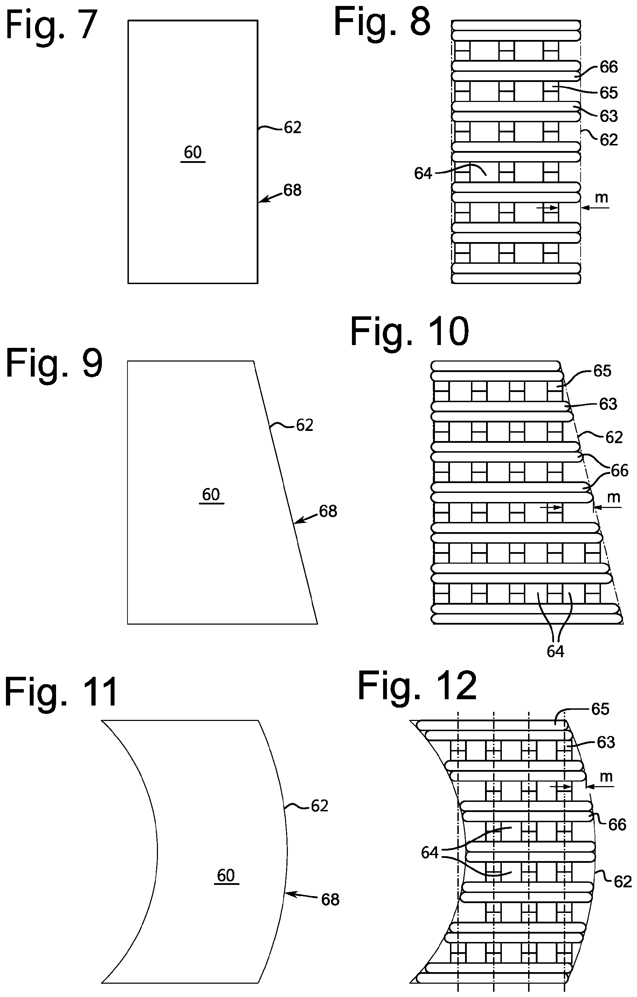

Figures 7, 9 and 11 show examples of bodies having a volume 60 and a first side 62. The first side may, for instance, be straight (Fig. 7), slanted (Fig. 9) or curved (Fig. 11).

Referring to Figures 8, 10 and 12, when an infill structure based on repeated unit cells is deployed in the volume 60, the orthogonal repetition of unit cells 64 may cause a problem.

Referring to Fig. 8, even when applying an infill structure to fill a body having straight, orthogonal sides, a distance between opposite walls may exceed an exact integer number of
unit cells 64. As a result, slicing and printing the respective body using the unit cells 64 may result in loose ends 66 of the clipped units. Herein, said ends 66 have an unsupported length m.

The structure may include first lines 63 extending in a first direction, and second lines 65 extending in a second direction. For a typical repetitive unit cell structure, the second direction may be perpendicular to the first direction. Unsupported length m herein relates to a length of the first lines 63 between an outer surface of the volume 60 and a first one of a number of second lines 65.

A similar problem may arise when slicing and printing a porous body having a slanted first wall 62 (Fig. 10) or a curved first wall 62 (Fig. 12). Herein, the fixed size and shape of the unit cells 64 inherently leads to sections of the first wall wherein loose ends 62 may occur.

Said loose ends 66 on the outside of the porous body are typically unsupported by an underlying structure, such as the second lines 65, and are therefore not printed accurately. Also, the ends 66 are typically relatively rough and may deform when the body is loaded. If the ends 66 are comprised in a porous structure of a medical implant, the ends 66 may harm tissue during insertion in the body of a patient, or the forces of insertion may damage the loose ends, potentially harming the patient.

Referring to Figure 3, some slicers add perimeter lines 68 to an infill structure. The perimeter lines 68 obviate loose ends. However, at the same time the perimeter lines 68 form a closed outer surface and thereby significantly reduce the intended (surface) porosity of the respective porous body. This same solution is used in, for instance, the methods disclosed in US11135771 and US11481886, as referenced in the introduction.

Of course the same problem exists for infills that are not necessarily based on a repeating unit cell. In general, loose ends may occur in any object in which a predefined infill structure, for instance of any of the above-described types, does not perfectly align with the circumference of the infill area so that clipping occurs and loose ends would be produced. This effect is particularly pronounced in infill structures that follow a predictable pattern. In these infill structures, it is possible to predict from e.g. an internal section of the infill area the type of infill and how it would extend to the circumference of the infill area, and that accordingly loose ends would be created. According to the invention however, the (porous) infill structure is adapted, i.e. changed from its predicted occurrence, to match the circumference. This adaptation is apparent from a deviation in the infill structure, i.e. an adaptation of the infill structure at the circumference of the infill area from the infill structure predicted from the pattern.

In general, a predictable pattern for an infill can be any one of the known infill structures that depending on the pattern prescribe definitively the infill structure as a whole once from a certain part of the infill it has been determined which pattern the infill structure follows. As an example, and as described above, an otherwise repetitive pattern may be adapted to the circumference. In particular, repeating unit cells are predicted to repeat further, as the repeating unit cell is a known pattern for infill structures. As further examples, reference is made to points i) - x) above.

Generally referring to Figures 13, 14 and 15, the method of the present disclosure may take an infill structure having unit cells 64. Herein, the method includes a step of defining a circumference 68 of the respective body 60. The body referred to defines the area to be provided with a porous infill structure, also referred to as the infill area or infill volume. Next, the method adapts the porous infill pattern to the circumference 68. Finally, the method includes filling the volume of the respective body with the adapted infill structure.

For a body having straight edges, as exemplified in Fig. 7, the size of the unit cells 64 may be adapted. See Fig. 13. For a body having a slanted wall, respective unit cells 64 may be adapted to provide a best fit. See Fig. 14. As shown in Fig. 15, for a curved first surface 62, the pattern including the unit cells 64 may be adapted to the specific curvature, in one or more directions, of the surface 62.

As exemplified in Figures 13, 14, and 15, the adapted infill structure of the disclosure obviates loose ends. Herein, boundaries of respective unit cells 64 follow the circumference 68 of the respective body.

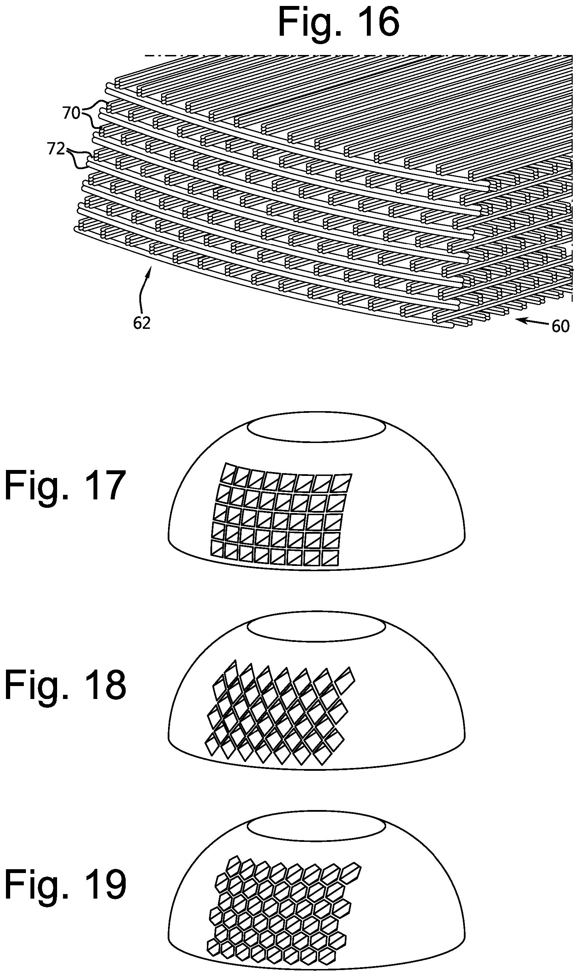

Figure 16 exemplifies a printed porous body 60, comprising five layers of a first pattern 70 and two layers of a second pattern 72, printed intermittently. Herein, the tracks or lines of the second pattern 72 have been adapted to follow, or conform to, the circumference of the curved surface 62 of the body 60. At the perimeter or circumference of the curved surface 62, said tracks of the second pattern 72 support ends of the tracks of the first pattern 70.

Figure 12 exemplifies a printed porous body 60, comprising three layers of a first pattern 70 and two layers of a second pattern 72, printed intermittently. Herein, the tracks or lines of the second pattern 72 have been adapted to follow, or conform to, the circumference of respective surfaces of the body 60, including the curved first surface 62. Due to the adaptation of the infill structure according to the method of the disclosure, at the perimeter or circumference of the curved surface 62, said tracks of the second pattern 72 support ends of the tracks of the first pattern 70.

Thus, the method of the present disclosure obviates loose ends of tracks at the outer surface of a porous body, while at the same time maintaining the intended porosity. Said porosity is typically determined by the respective infill pattern and the pore size on the outer surface of the body. The method of the disclosure obviates limiting said porosity. At the same time, the method of the disclosure enables to print porous bodies having a relatively smooth outer surface without loose ends.

A smooth structure is beneficial since it is robust, and avoids harm to the tissue when introduced in a patient. Also, the smooth outer surface is aesthetically pleasing. An infill structure according to a method of the disclosure may be designed to have lines in multiple directions along the outer surface of the porous structure. Herein, as shown in the figures, the method of the disclosure allows the infill structures to be adapted to a double-curved surface. Figures 17, 18 and 19 show respective examples of porous bodies providing a set porosity while at the same time providing a smooth outer surface. Figure 17 shows a plus-shaped pattern; Figure 18 shows an X-shaped pattern; and Figure 19 shows a hexagon pattern. These are just some examples, many more shapes are possible, even irregularly shaped organic patterns mimicking the internal structure of trabecular bone.

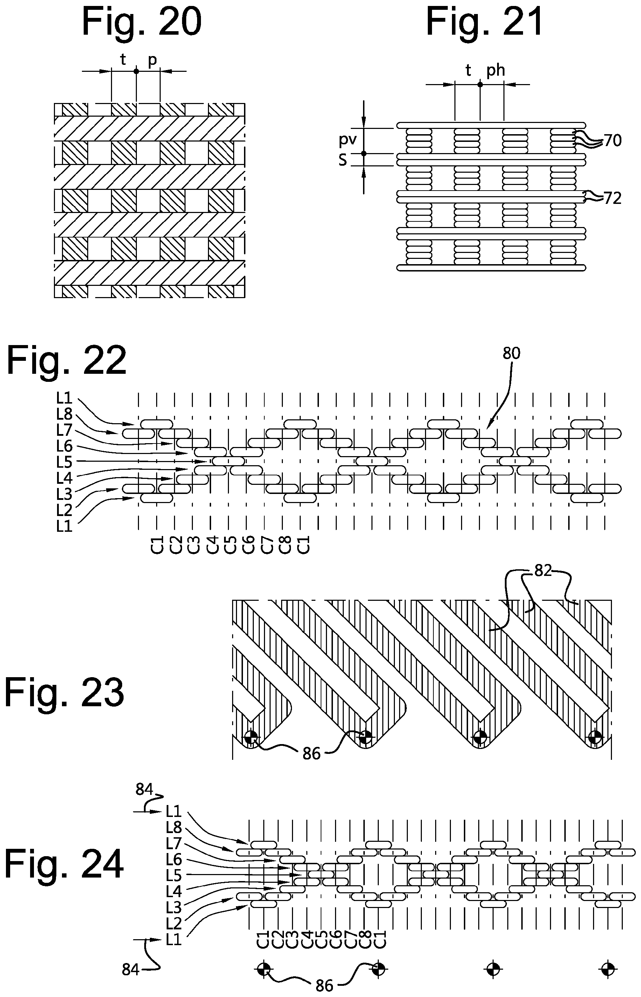

Generally referring to Figures 20 and 21, the width of printed tracks is primarily determined by the diameter of the nozzle. The layer height can be selected from a range limited by the capabilities of the printing process. Typically, the layer height is set as an optimum between print time and roughness of the printed product. The dimensions of pores in the infill structure can be chosen relatively freely. Pores herein are openings between respective tracks of a pattern.

As an example, the dimensions of a lattice structure in a horizontal plane (parallel to the build plate of the printer) is defined by track width t and pore size p.

A lattice structure seen from the side is determined by pore size track width t and pore size pₕ. Vertical pore size pᵥ and the thickness s of the bar are determined by layer height h and the number of layers 70 of the first pattern printed in between layers 72 of the second pattern.

In a practical embodiment, the layer height may be, for instance, exceeding 0.05 mm, for instance 0.1 mm, for instance 0.15 mm. The layer height may be smaller than, for instance, about 1 mm, for instance about 0.5 mm, for instance about 0.3 mm. In a practical embodiment, the layer height may be about 0.05, 0.1, 0.15, 0.2, 0.25, 0.3 mm.

In a practical embodiment, the track width may be about 0.2 mm or more, for instance about 0.3 mm, 0.36 mm, 0.4 mm, 0.45, 0.5 mm or more. The track width may be in the order of 5 mm, 3 mm, 2 mm, 1.5 mm, 1.2 mm, 1 mm, or less. The track width may be, for instance, about 0.3, 0.36, 0.45, 0.7, 1.0, or 1.2 mm.

The width of respective tracks can vary depending on the infill structure needed. For porous areas, typically the nominal track width is printed.

The track width is typically in the range of about 1 to 10 times the layer height. For instance, the track width is at least about 1.2 times the layer height. The track width may be about 10 times, for instance about 8 times, for instance about 6 times, the layer height or less.

In a practical embodiment, to provide an aesthetically pleasing printed object, the pattern on the right (side view in Figure 21) can be selected to be substantially similar to the pattern on the left (the top view of Figure 20). For instance: s ≈ t and pᵥ ≈ pₕ ≈ p.

Thus, if the lattice structure consists of multiple types of layers, the dimensions of the lattice are determined by the number of times each layer type is repeated as part of a sequence.

As an example, it is typically possible to define various sequences of layers. For instance, an infill may have an A - B - A - C kind of sequence. Herein, vertical pillars formed using the B-pattern can be positioned having a horizontal offset with respect to the position of vertical pillars due to the C-pattern of the infill structure.

Figures 22 to 36 provide exemplary steps to create the X-shaped pattern as shown in Figure 18. The pattern can be projected on a curved or double-curved surface using one of, or a combination of, the embodiments as described herein below.

To explain the basic idea, the pattern 80 may be drawn as if it is created in a traditional orthogonal grid (Fig. 22). In Figure 22, the vertical axis indicates respective layers. The example of Fig. 22 comprises eight layers, ranging from layer L1 to L8. The horizontal axis in Fig. 22 represents horizontal position, represented by, for example, eight columns ranging from column C1 to C8. The number of columns and the number of layers per subsection of the pattern 80 can be varied within a certain range, to change the width and height of the pattern.

In the example of Figure 22, an X-pattern 80 can be created on the side of the porous structure. Herein, the grid is repeated every eight layers. As indicated in Fig. 22:
- the tracks of layer L1 leave the front surface of the porous area in column C1.
- the tracks in layer L2 leave the front surface of the porous area in columns C2 and C8.
- the tracks in layer L2 leave the front surface of the porous area in columns C3 and C7.
- etc.

The structure is, for example, repeated every eight columns C1 to C8.

Figures 23 and 24 exemplify the printing of the first layer L1. Fig. 19 indicates a top view of track pattern 82 related to layer L1. A front view of the layers is displayed in Fig. 24, wherein the front view related to the first layer L1 is indicated by arrows 84.

In the top view of Fig. 23, the target points 86 indicate where column C1 meets the outer surface of the porous body. The start of the tracks in each respective layer L1 to L8 may be oriented in 3D relative to these target points.

The target points 86 indicate where the second column C2 meets the outer surface of the porous body.

In the example of Fig. 23, the tracks in layer L1 leave the surface of the respective body at an angle of about 135°. The tracks in the second layer L2 leave under an angle of 45°. In each subsequent layer, the angle under which the tracks leave the curved surface is swapped. Other sequences are also possible, for instance wherein the angle is swapped for instance every three or four layers.

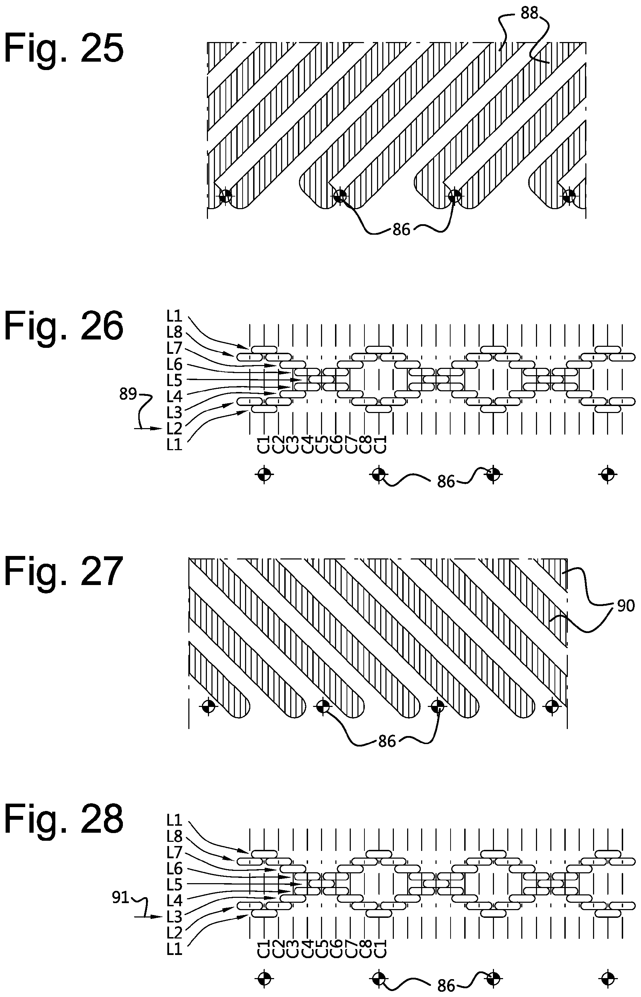

The second layer L2 is printed as exemplified in Fig. 25. Fig. 25 indicates a top view of second track pattern 88 related to the second layer L2. Figure 26 indicates the front view of the respective layers at the outer surface of the body. The arrow 89 indicates the front view of the second layer L2.

The third layer L3 may be printed as exemplified in Fig. 27. Fig. 27 indicates a top view of third track pattern 90 related to the third layer L3. Figure 28 indicates the front view of the respective layers at the outer surface of the body. The arrow 91 indicates the front view of the third layer L3.

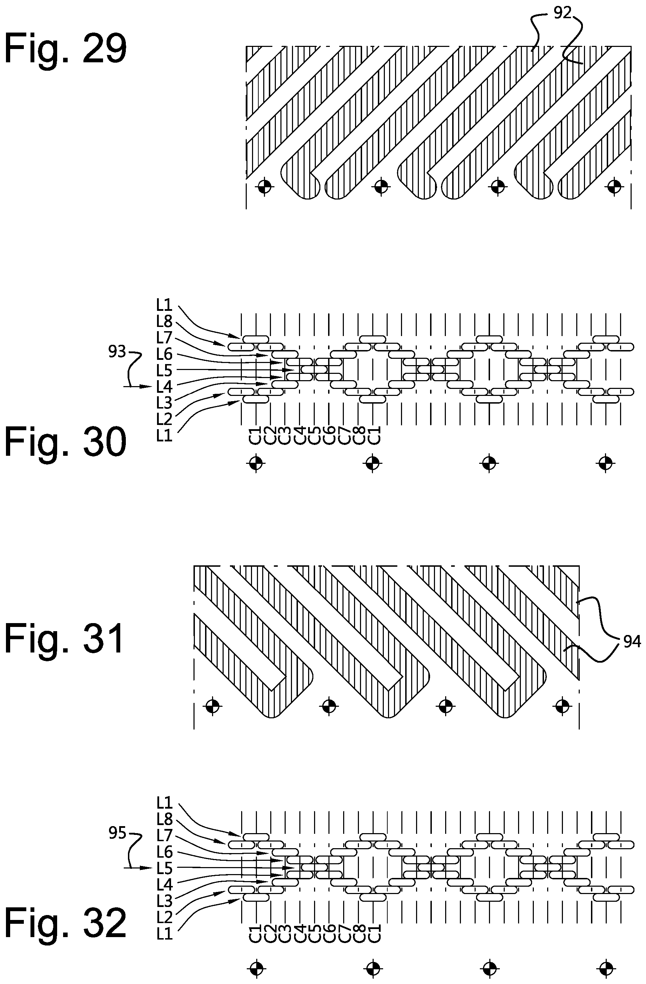

The fourth layer L4 may be printed as exemplified in Fig. 29. Fig. 29 indicates a top view of fourth track pattern 92 related to the fourth layer L4. Figure 30 indicates the front view of the respective layers at the outer surface of the body. The arrow 93 indicates the front view of the fourth layer L4.

The fifth layer L5 may be printed as exemplified in Fig. 31. Fig. 31 indicates a top view of fifth track pattern 94 related to the fifth layer L5. Figure 32 indicates the front view of the respective layers at the outer surface of the body. The arrow 95 indicates the front view of the fifth layer L5.

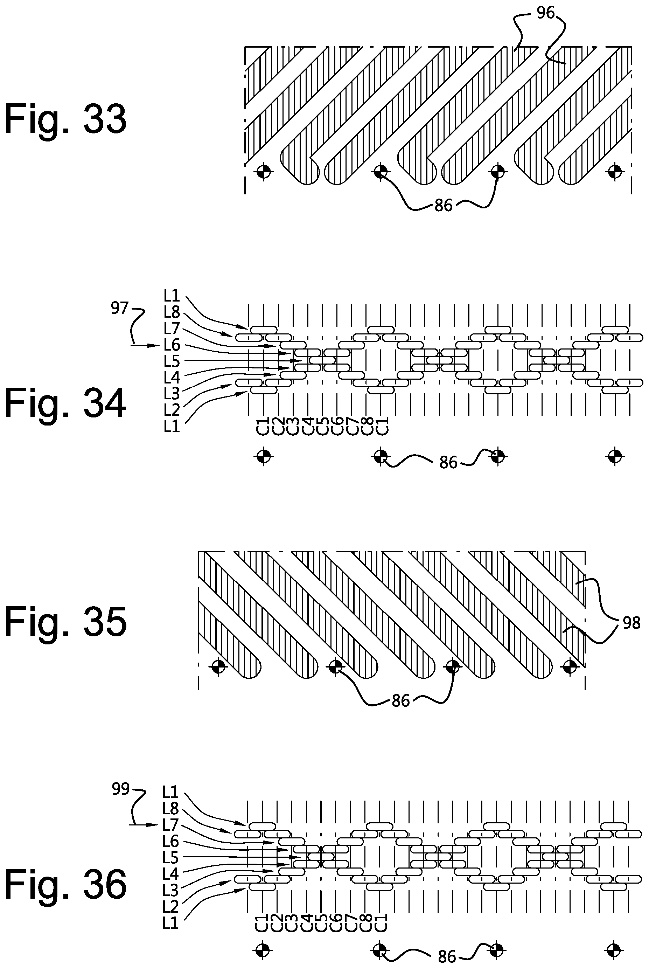

The sixth layer L6 may be printed as exemplified in Fig. 33. Fig. 33 indicates a top view of sixth track pattern 96 related to the sixth layer L6. Figure 34 indicates the front view of the respective layers at the outer surface of the body. The arrow 97 indicates the front view of the sixth layer L6.

The seventh layer L7 may be printed as exemplified in Fig. 35. Fig. 35 indicates a top view of seventh track pattern 98 related to the seventh layer L7. Figure 36 indicates the front view of the respective layers at the outer surface of the body. The arrow 99 indicates the front view of the seventh layer L7.

Note that in this example, the pattern 98 of the seventh layer L7 is the same as the pattern 90 of the third layer L3. Likewise, a track pattern of the eighth layer L8 (not shown) may be similar to the track pattern 88 of the second layer L2 (see Fig. 26).

Throughout figures 23 to 36, the target points 86 indicate where the tracks of the respective columns C1 to C8 meet the outer surface of the respective porous body.

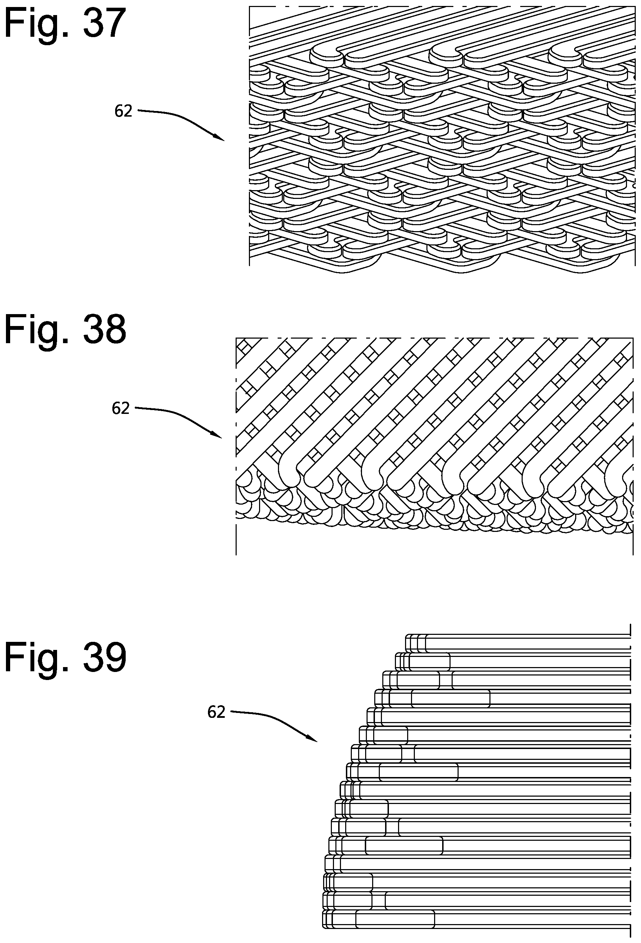

As explained with respect to various embodiments of the disclosure, the pattern may be mapped to the (double) curved surface 62 of a respective body. Figures 37, 38 and 39 show examples of a front view, top view, and side view of such curved surface, provided with the X-shaped pattern using the track patterns of Figures 23 to 36. Referring to Figures 37 and 38, note that the curved surface as printed using a method according to the present disclosure has the same porosity as the selected infill structure. Added lines 68 on the circumference of the curved surface 62, as exemplified in Fig. 3, are obviated.

Three different embodiments of methods according to the disclosure are described herein below. Each embodiment has its own advantages and complexity.

As mentioned before, a typical infill structure may comprise multiple patterns, repeated in a sequence of choice over different layers. For instance, an infill structure may repeat two patterns A and B, in a sequence of, for example, A - A - A - B - B - etc. Herein, please note that the first pattern A and the second pattern B may be handled using different embodiments of a method according to the present disclosure. In general, the direction of tracks may be the same for all layers of type A, and for all layers of type B. However, the exact locations of the tracks may be different for the individual layers of types A and B respectively.

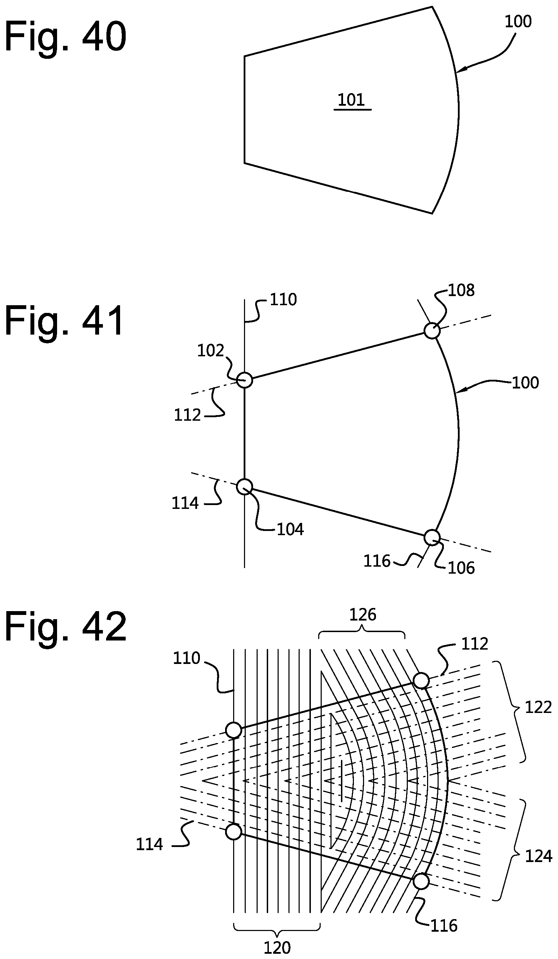

Generally referring to Figures 40 to 44, in a first embodiment, the method may comprise the steps of:
1. Providing a print job comprising at least one object;
2. Each object is comprised of at least one body;
3. For each body, print settings can be defined. Print settings may include, for instance, a selection of an infill structure for each body. Also, print settings may indicate whether a specific body is to be printed including a perimeter line, or without a perimeter line (for a porous body);
4. Each body is sliced in one or more slices;
5. For every slice, the area is defined that is to be filled with a porous structure.

As referenced above, see for instance Figures 2 to 6, an object may comprise multiple bodies each having different printer settings. Figure 40 shows a top view of an exemplary perimeter 100 of a slice of a porous body. The respective slice has an area 101. A porous body will be printed without a perimeter line, and the area 101 will be filled with a porous infill structure.

Generally referring to Figure 41, the method of the disclosure may include a step of identifying corner points 102, 104, 106, 108 of the perimeter line 100.

Subsequently, the corner points are connected using lines 110, 112, 114, 116 extending along the side of the slice area 100 of the body. Herein, said lines extend beyond the corner points 102, 104, 106, 108. The lines 110, 112, 114, 116 extend along the perimeter 100 of the slice area of the respective body.

Generally referring to Figure 42, next the method creates concentric offsets 120, 122, 124, 126 of the sides by creating offsets of the lines 110, 112, 114, 116 including the extensions, towards the inside of the body. Said offset of the lines 110, 112, 114, 116 have a pitch, i.e. a distance between respective copies of the lines. Said pitch corresponds with the desired porosity of the porous infill structure as selected.

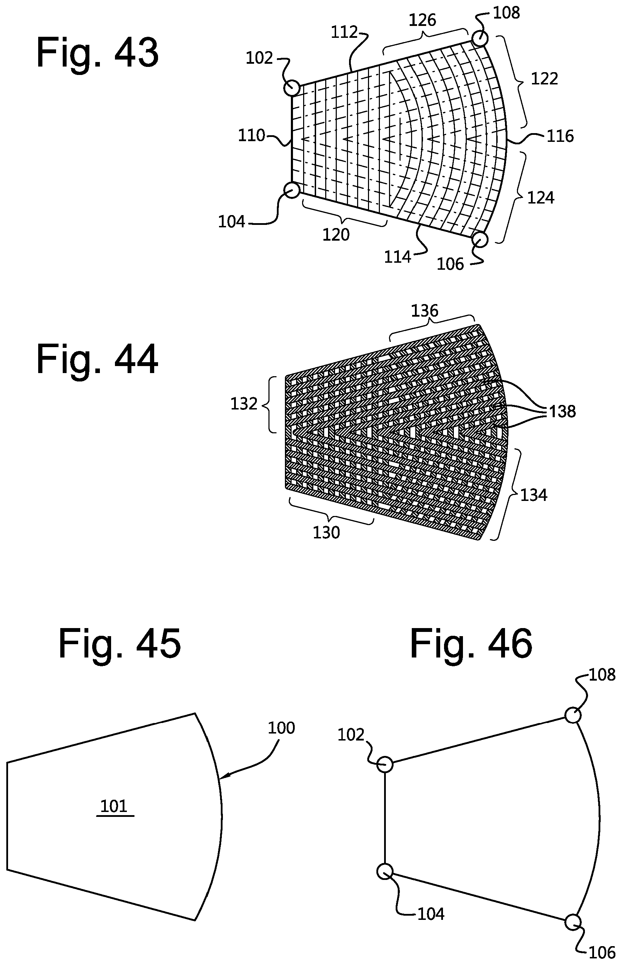

Referring to Figure 43, in a next step, the lines 110, 112, 114, 116 and the concentric offsets 120, 122, 124, 126 are trimmed to the area of the slice of the body. Trimming herein means the parts of the respective lines and offsets extending beyond the perimeter line 100 are deleted.

For printing, the offsets of one subset of sides may be printed in one type of layer (for instance, a layer for printing a first pattern A), while the offsets of another subset of sides may be printed in another layer (for instance, a layer for printing a second pattern B).

In the example of Figure 43, for instance the offsets 120 and 126 may be assigned to the first pattern A. The offsets 122, 124 may be assigned to the second pattern B.

The same steps are repeated to other slices of the respective body.

The resulting pattern, as shown in Fig. 43, is converted to print instructions. The print instructions include instructions for all slices in the body. The print instructions are subsequently supplied to a 3D printer for printing.

Figure 44 shows a final structure of a printed pattern of tracks 138. The structure includes respective tracks 130, 132, 134 and 136. The tracks correspond to the offsets 120, 122, 124 and 126 respectively.

In the example of Figure 44, the tracks 130 and 136 are printed in a first patterns of a first layer. The tracks 132 and 134 are printed in a second pattern of another layer. The first pattern and the second pattern are printed intermittently, as described more generally herein above.

Generally referring to Figures 45 to 50, in a second embodiment, a method according to the disclosure may include the steps of:
1. Providing a print job comprising at least one object;
2. Each object is comprised of at least one body;
3. Define print settings for each body. Herein, the user can define settings (such as infill structures) for printing each respective body;
4. Dividing each body in slices. Alternatively, this may be referred to as slicing the respective at least one body. Each body is divided in at least two slices;
5. Defining for every slice an area that is to be filled with a porous infill structure.

Generally referring to Figure 45, in a first step, the method determines an area 101 of a slice of a body. The slice has perimeter 100.

Referring to Figure 46, in a next step, the method identifies corner points 102, 104, 106, 108 of the circumference 100.

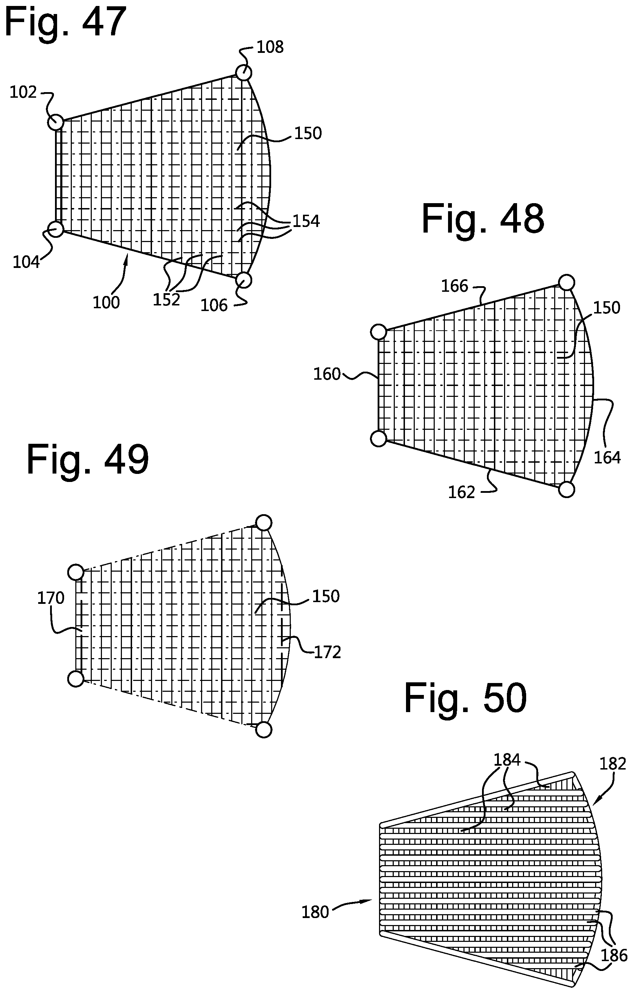

Referring to Figure 47, subsequently, the method fills the area 101 with an infill structure 150.

In a practical embodiment, the infill structure 150 may be repetitive, based on a unit cell which is repeated in an orthogonal grid. The repetition of the respective unit cell can be set within a range, and may be referred to as the pitch of the infill.

In the example of Figure 47, the infill structure is a rectilinear infill structure. Herein, first lines 152 extending in a first direction may be assigned to a first pattern A. Second lines 154 extending in a second direction may be assigned to a second pattern B. The first and second patterns may be printed in different, intermittent layers.

In a next step, see Figure 48, additional lines 160, 162, 164, 166 are added to the infill structure 150. The additional lines follow the circumference 100 of the area 101. The additional lines 100 to 166 correspond to instructions for the printing of tracks along the circumference 100 of the area 101.

Next, see Figure 49, superfluous lines 170, 172 are removed. Herein, lines or tracks can be considered to be superfluous if certain unit cells become smaller than the pitch as set for the respective infill structure. In the example of Figure 49, the unit cells on the outside, i.e. near the newly added tracks 160 and 164, are smaller than the pitch of the other unit cells. In other words, the superfluous lines 170, 172 correspond to tracks which are too close, i.e. within the set pitch, to the tracks 160, 164 at the edges of the area 101. By removing the superfluous lines 170 and 172, all unit cells of the infill structure 150 are equal to or exceed the size of a unit cell corresponding to the selected pitch.

Optionally, original pattern 150 can be moved (in the plane of Figure 49). Herein, the algorithm may include a step which optimizes the location of the pattern in a way that aims to avoid removal of superfluous tracks.

The same steps are repeated for other slices of the respective body.

The resulting pattern of tracks, as shown in Fig. 49, is converted to print instructions. The print instructions include instructions for all slices of the respective body. The print instructions are subsequently supplied to a 3D printer for printing.

Figure 50 shows a final structure 180 of a printed pattern of tracks 182. In the example of Figure 50, the printed tracks 182 include first tracks 184 printed in a first pattern of a first layer, and second tracks 186 printed in a second pattern of a second layer. As an example, the first tracks 184 may substantially extend in a first direction and the second tracks 186 may substantially extend in another direction.

The first pattern and the second pattern are printed intermittently, as described more generally herein above.

The second embodiment is relatively simple and easy to implement, while still offering many of the advantages of the first embodiment. In addition, the second embodiment enables to create a see-through porous structure. Such structure may be perceived positively by some users, such as doctors, since they can visually confirm the structure is fully open and clean. On the other hand, some of the pores in the structure exceed the set pitch of the design intent.

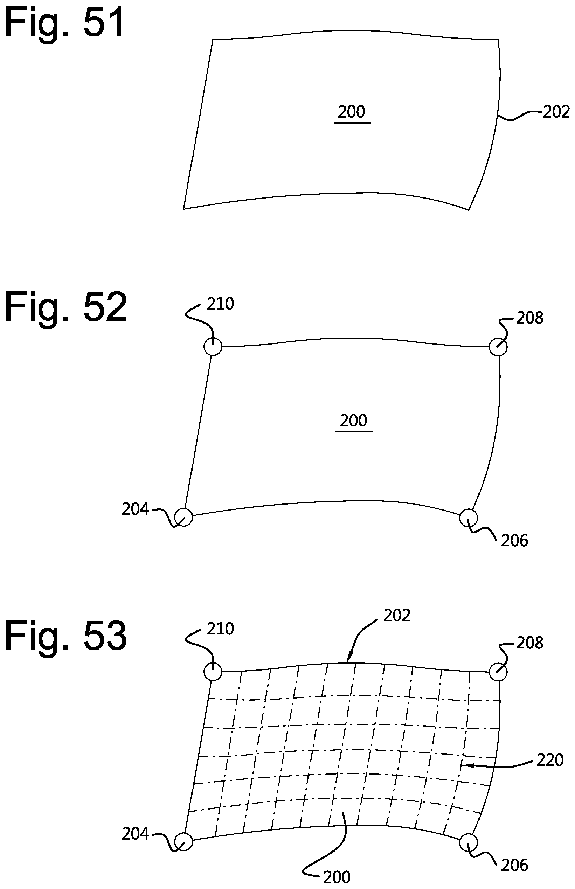

Generally referring to Figures 51 to 56, in yet another embodiment of a method, a virtual non-orthogonal XY grid is created. Thus, the method is suitable for both unit cell based infill structures and other types of infill structures (for instance three dimensional infills, such as a gyroid structure).

The method includes the steps of:
1. Providing a print job comprising at least one object;
2. Each object is comprised of at least one body;
3. For each body, settings are defined (such as infill structures) for printing;
4. Slicing each body. Herein, each body may be divided in at least two slices.

Generally referring to Figure 51, for every slice, an area 200 having a perimeter or circumference 202 is defined. The area 200 is to be filled with a porous structure.

In a next step the method includes identifying corner points of a virtual grid. Figure 52 shows a top view of the area 200 including respective corner points 204, 206, 208, 210.

In a practical embodiment, other infill structures are possible, such as a triangular infill or an infill having a less regular pattern than a grid are possible. As a result, the number of corners may differ from the four points as exemplified in Fig. 52. For a body without sharp corners (sharp corners having an angle lower than 90 degrees; such as the example of Fig. 52), it may be possible to select a corner that divides an edge of the body in equal sections, or that generates the nicest (for instance, most perpendicular) grid.

Subsequently, the method includes the step of projecting a virtual grid 220 onto the area 200 to be filled with the infill structure. See Figure 53.

The virtual grid 220 may, for example, follow the circumference 202 of the area 200. Herein, a virtual grid may be created by dividing a rectilinear grid over the available area 200. The virtual grid lines of the virtual grid follow the shape of the circumference 202. A pitch of the original rectilinear grid herein may drive the division of the grid over the area 200. The latter may indicate that the size of a unit cell 224 of the virtual grid is equal to or exceeds the size of the unit cells of the original rectilinear grid.

In a subsequent step, an infill structure is projected onto the virtual grid.

When an infill structure based on a regularly repeated unit cell is projected onto the virtual grid, the sides of each unit cell of the infill will follow the sides of the unit cells 224 and the corresponding grid lines of the virtual grid. As exemplified in Fig. 53, said virtual grid lines may deviate from rectilinear. For instance, the virtual grid lines may be curved.

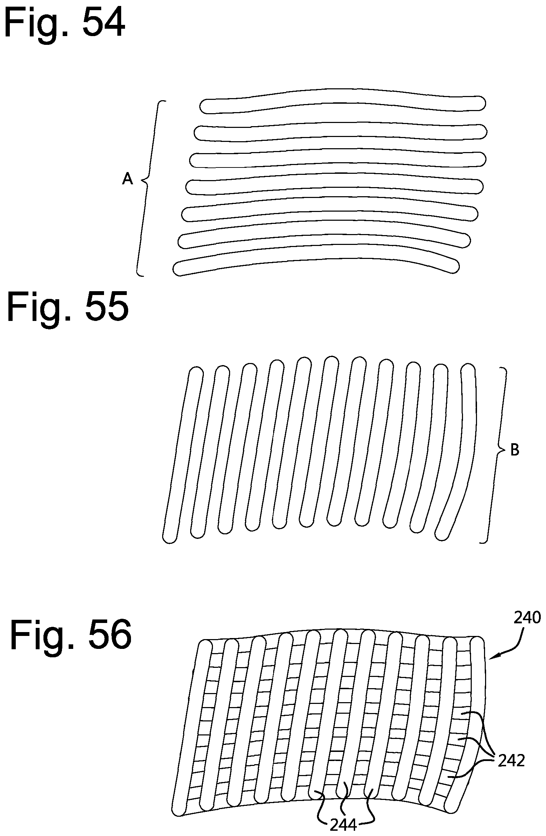

Projecting the infill structure onto the virtual grid is exemplified, in Figures 54 to 56, based on an infill having two patterns, for example first pattern A and second pattern B. For each layer of the porous infill structure, the respective infill pattern is projected on the virtual grid. Figure 54 shows the first infill pattern A projected on the virtual grid 220 of Fig. 53. Figure 55 shows the second infill pattern B projected on the virtual grid 220 of Fig. 53.

Please note that the examples of Figures 54 and 55 concern patterns A and B, which are originally based on a rectilinear type grid. However, any infill pattern can be projected on the virtual grid 220. For instance, a gyroid infill structure or another irregular infill structure based on a function of the vertical axis can be selected and projected on, or in other words adapted to, the virtual grid.

Figure 56 shows a top view of a three dimensional structure 240 of printed tracks 242, 244, corresponding to the projected first and second patterns A and B respectively.

If the area to be filled is non-rectangular, as exemplified in Figures 51 to 56, the local distance between tracks will vary. In the slicer, the intended distance between adjacent tracks can be defined. For instance, a range of the distance between the tracks can be set, including a minimum and/or a maximum intermediate distance between tracks. Thus, the method of the disclosure enables control of the size of pores of the infill. Said size is defined by, for instance, the size of respective unit cells.

In a practical embodiment, various options can be implemented to meet the intent with respect to porosity and/or pore size. For instance, upon projecting the infill on the virtual grid:
1. the average distance between tracks is set to meet the pitch and intent as near as possible within each layer.
2. the average distance between ends of the infill pattern at the boundaries of the area 200 facing the outside of the body is optimized to meet the intent as near as possible.
3. the number of tracks can be modified within the infill structure to optimize the average distance (for instance, for strongly wedge-shaped sections of a slice of a body).

As near as possible herein may include within a set minimum or maximum threshold.

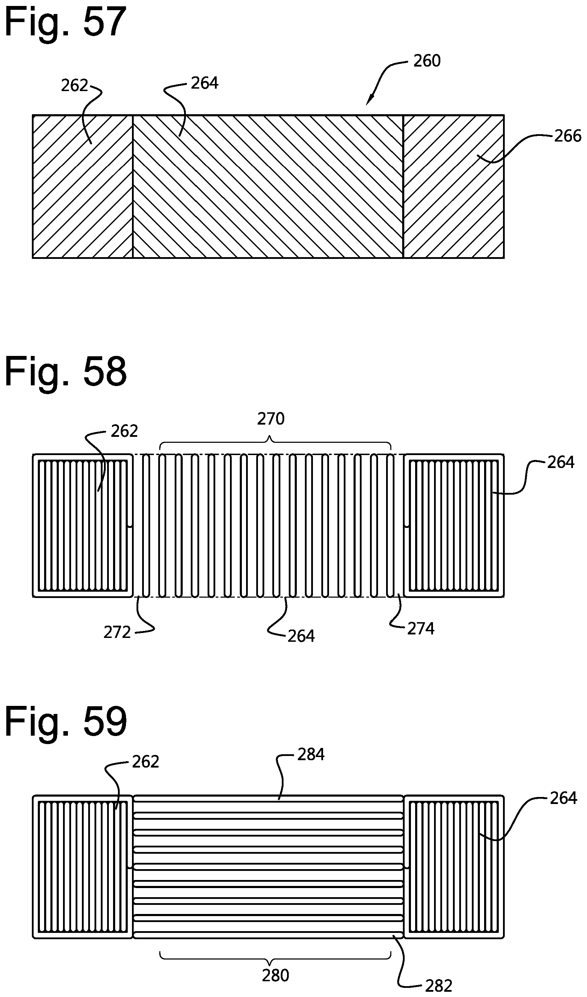

Generally referring to Figure 57, an exemplary object 260 may be comprised of three bodies 262, 264, 266. Herein, the bodies 262 and 266 on the outside of the object may have to be printed non-porous, including a closed perimeter. The body 264 in the middle may have to be printed as a porous structure, with an open perimeter.

In a practical embodiment, on sides that are adjacent to the solid bodies 262, 266, an infill pattern 270 of the porous body 264 may start with a pore 272, 274 (see Figure 58). On sides that are adjacent to open areas, the repeating pattern 280 may start with a track 282, 284 (se Figure 59).

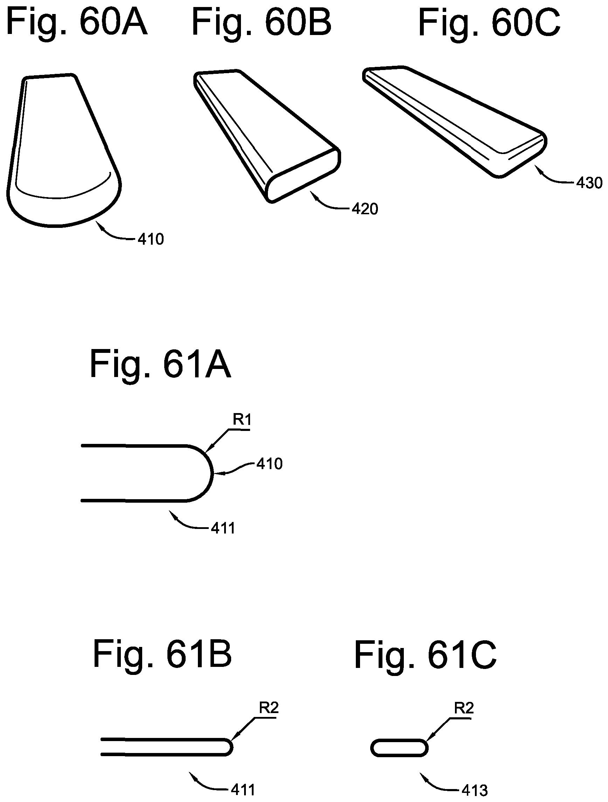

Figures 60A to 63C schematically exemplify some of the differences between porous bodies printed using a method of the present disclosure, compared to porous bodies created using conventional methods.

Figures 60A and 61A to 61C indicate an end 410 of a track printed using a method of the present disclosure. Said end 410 is relatively smooth. Relatively smooth herein can be observed, for instance, due to a radius R1 of the end 410 in top view. Said radius R1 is relatively large, for instance exceeding 0.35 times the track width. Radius R1 may be, for instance, in the order of 0.4, 0.42, 0.44, 0.45, 0.46, 0.50, 0.53, 0.55, 0.57, 0.6., 0.64, 0.65, 0.68, 0.7 times the track width or more. Track width herein relates to a width of the track 413.

In an embodiment, radius R1 is in the range of about 0.3 to 0.85 times the track width. Radius R1 may be in the range of about 0.35 to 0.8 times the track width. Radius R1 may be in the range of about 0.4 to 0.7 times the track width. Radius R1 may be in the range of about 0.42 to 0.65 times the track width. Radius R1 may be in the range of about 0.45 to 0.6 times the track width.

Figure 60B shows an end 420 of a 3D printed track of an exemplary porous body created using a conventional method. First, tracks of the exemplary porous body are 3D printed using a conventional printing method. As conventional 3D printing methods typically provide relatively solid bodies, i.e. bodies having an outer surface having a relatively limited porosity, part of the outer surface may be removed to increase the porosity. For instance, the outer surface may be machined, for instance by etching, milling, sawing, or sanding.

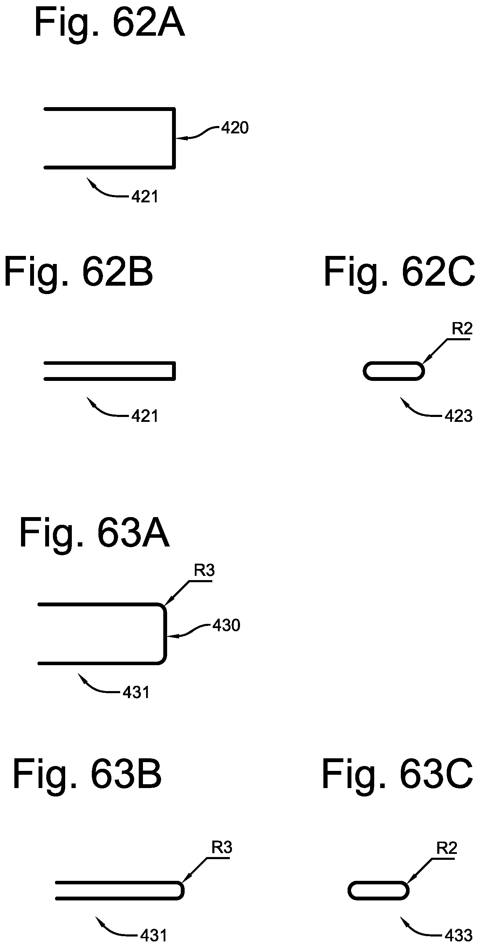

As exemplified in Fig. 62C, a radius R2 of the sides of the track 423 may be substantially similar to the sides of the track 413 (Fig. 61C) as printed using the method of the disclosure. Due to the machining step however, the machined end 420 becomes relatively rough (Fig. 62A). Relatively rough herein means, for instance, virtually straight. Potentially, said end 420 has irregularities, such as relatively sharp protrusions or burrs (not shown). Although an object comprising a porous body produced as described above and having ends 420, such ends may exceed roughness or sharpness thresholds for many practical applications. In particular, for medical applications wherein the 3D printed object is intended to be inserted in the body of a living being, the end 420 may be unsuitable.

Figure 60C shows an end 430 of a 3D printed track of an exemplary porous body created using another conventional method. Herein, an exemplary porous body can be 3D printed using a conventional printing method. Subsequently, part of the outer surface may be removed to increase the porosity. For instance, the outer surface may be machined, for instance by etching, milling, sawing, or sanding. Subsequently the outer surface of the respective porous body may be treated to reduce the roughness of and remove sharp protrusions from the end 430. The treatment step may include, for instance, a heat treatment step such as baking, burning, working using a torch or flame. As a result of the heat treatment, the end may be provided with smoother corners, having for instance a radius R3 (Fig. 63A). However, in practice, the radius R3 will be significantly smaller than the radius R1 (Fig. 61A) which can be achieved when creating a porous body using a method of the present disclosure.

As outlined above, objects having at least one porous body that are printed using a method of the present disclosure will have distinguishable characteristics with respect to objects printed using conventional 3D printing methods. The porous sections of the object will be relatively smooth and visually appealing. The smoothness can be observed both visually and sensory.

Herein, a porous object has at least two bodies, of which at least one body is printed with a porous infill structure extending to the outer surface of the respective object. In an object according to the present disclosure, ends of the porous infill structure near and extending towards said outer surface are supported by other tracks. Herein, the outer surface still has a porosity exceeding a preset threshold. Said threshold may be, for instance, a porosity exceeding 40%, 43%, 45%, 48%, 50%, 55%, 60%, 65%, 70%, or 75%. In an embodiment, a majority of the tracks of the porous infill structure extending towards the outer surface of the object are supported by other tracks. For instance, a majority of first tracks of the porous infill structure extending towards the outer surface of the object are supported by second tracks extending in a direction substantially perpendicular to a direction of the first tracks.

In a practical embodiment, an unsupported length m of the first tracks is at most 1.99, 1.8, 1.69, 1.5, 1.4, 1.39, 1.2, 1.19, 1, 0.99, 0.9, 0.84, 0.8, 0.7, 0.69, 0.65, 0.6, 0.59, 0.55, 0.5, 0.49 times the track width of said first tracks.

In a practical embodiment, at least 50%, 51%, 55%, 60%, 61%, 63%, 65%, 70%, 71%, 75%, 80%, 81%, 91% of the ends 410 of the first tracks 413 are supported by other tracks, preferably the second tracks, near the porous section of the outer surface of the object.

In a practical embodiment, the porous section of the outer surface of the object is printed with tracks having spacings in between respective tracks. Said spacing provide openings or passages to the internal volume of the object, thereby providing the porosity. In an embodiment, the spacings are at least 0.5, 0.4, 0.3, 0.2, or 0,1 times the width of the tracks 413.

Objects comprising at least one porous body printed using a method according to the present disclosure are suitable for a wide range of applications. For instance, the methods are suitable to 3D print filters, such as exemplified in Figure 6. Due to the relatively smoothness and rounded curvature R1 of free ends 410 of the objects printed using a method of the disclosure, said objects are also suitable for medical applications, as implantable device. Medical implantable applications include, but are not limited to, for instance:
- Craniomaxillofacial applications, such as cranial implants (parts of the skull) and jaw implants;
- Trauma plates (for instance for collar bones and extremities);
- Cervical implants (spine implants in the neck);
- Lumbar interbody fusion implants (spine, thorax region and lower back);
- Hip cups;
- Knee implants, such as femoral parts; and
- Ankle plates.

When considering fluid filtration, filters play a crucial role in eliminating impurities. Utilizing PEEK offers several advantages, making it an optimal choice for filter construction due to its chemical resistance, suitability for high-temperature applications, and robust mechanical strength. Through the application of 3D printing technology, filters can be designed with intricate shapes, providing distinct advantages in preventing dead zones in fluid flow, averting cavitation, and facilitating proper venting post-installation or servicing. The absence of dead zones and effective venting is particularly beneficial in preventing contamination nuclei, thereby enhancing the filter's service life. Furthermore, the mechanical strength of PEEK contributes to improved resistance against pressure and shear forces, enabling its utilization in higher-pressure or flow conditions for enhanced efficiency. This mechanical durability is advantageous not only during initial installation but also in the cleaning process, minimizing the risk of filter damage. To ensure the filter's efficacy, it is crucial to maintain smooth surfaces within the porous volumes, avoiding any loose tracks in these areas to prevent the generation of particles that could contaminate the fluid.

The methods of the present disclosure enable to 3D print porous bodies while obviating loose ends on the perimeter of the body, while also maintaining a set porosity. Although tracks are added around the perimeter of the porous body, said added tracks correspond to a respective pattern of a respective layer (for instance, first pattern A). The porosity of the printed structure is determined by the repetitive structure, i.e. the number of times the first pattern A is repeated with respect to a repetition of other patterns. For instance, if a porous body is printed with a repetition sequence of A-A-B-B-B-B-A-A etc., the selected porosity will not change due to the added lines at the circumference of patterns A or B.

Fused deposition modelling (FDM) may be used for extrusion based modelling. Herein, the modelling material may comprise a thermoplastic polymer composition. The thermoplastic polymer may be selected from polyethylene, polypropylene, ABS, polycarbonate, polyamide and polyarylether ketones (PAEK) like for example polyether ketone (PEK), polyether ether ketone (PEEK), polyether ketone ketone (PEKK), polyether ether ketone ketone (PEEKK) and polyether ether ketone ether ketone ketone (PEKEKK) and combinations thereof. The thermoplastic polymer composition may comprise at least 80 wt% of a PAEK, for instance at least 90 wt% of a PAEK. The thermoplastic polymer composition may comprise at least 80 wt% of a PEEK, for instance at least 90 wt% of a PEEK, for instance 100 wt% PEEK.

The printing material may also be enhanced with a substance that promotes bone ongrowth, such as, for example hydroxyapatite (HA). This substance may be mixed with the printing material prior to printing, or the printed object may be coated with this substance to obtain the effect.

Polyaryletherketones (PAEKs), such as the homopolymers polyetheretherketone (PEEK), polyetherketone (PEK) polyetherketoneketone (PEKK) or copolymers such as polyetherketone/polyetherdiphenyletherketone (PEEK/PEDEK) are high performance, semicrystalline thermoplastic polymers which have melting temperatures from 320°C to 400°C and are useful as matrix polymers for thermoplastic composites.

Other suitable thermoplastic polymers include polyethylenes, polypropylenes, polycarbonates, polyamides, polyethylene terephthalates, polyphenylene sulfides and polyetherimides.

A PAEK polymer in the context of the present disclosure may include one having a repeating unit of formula 1:

-O-Ph-(O-Ph)t1-CO-Ph-(O-Ph)w1-(CO-Ph)v1 (formula 1)

wherein t1 and w1 independently represent 0 or 1 and v1 represents 0, 1 or 2.

PAEKs, particularly including PEEK, can be manufactured by nucleophilic polycondensation of bisphenols with organic dihalide compounds in a suitable solvent in the presence of alkali metal carbonates and/or bicarbonates or alkaline earth metal carbonates and/or bicarbonates. Such processes are set out, for example, in patent documents EP0001879A, EP0182648A, EP0244167A and EP3049457A.

Preferably the PAEK has a crystallinity of at least 10%, more preferably at least 20%. However, for some applications a crystallinity such as less than 25% or less than 22% may be advantageous, particularly when the PAEK is to be extruded.

Crystallinity is measured by a DSC to determine the onset of Tg by the intersection of the lines drawn along the pre-transition baseline and a line drawn along the greatest slope obtained during the transition. The Tn is measured as the temperature at which the main peak of the cold crystallisation exotherm reaches a maximum. The Tm is the temperature at which the main peak of the melting endotherm reached a maximum. The heat of fusion for melting (ΔHm) is obtained by connecting the two points at which the melting endotherm deviates from the relatively straight baseline. The integrated area under the endotherm as a function of time yields the enthalpy (mJ) of the melting transition: the mass normalised heat of fusion is calculated by dividing the enthalpy by the mass of the specimen (J/g). The level of crystallisation (X(%)) is determined by dividing the heat of fusion of the specimen by the heat of fusion of a totally crystalline polymer, which for polyether ether ketone is 130J/g. ISO 11357-1 to ISO 11357-4 describes the test methodology used to determine said measurements.

A preferred PAEK is polyether ether ketone (PEEK), wherein the repeat units are wherein t1=1, v1=0 and w1=0.

Said polyaryletherketone suitably has a melt viscosity (MV) of at least 0.06 kNsm⁻², preferably has a MV of at least 0.09 kNsm⁻², more preferably at least 0.12 kNsm⁻². Said polyaryletherketone may have a MV of less than 1.00 kNsm⁻², preferably less than 0.5 kNsm⁻².

Said polyaryletherketone may have a MV in the range 0.09 to 0.5 kNsm⁻², preferably in the range 0.1 to 0.3 kNsm⁻², preferably having a MV in the range 0.1 to 0.2 kNsm⁻².

An MV of 0.15 kNsm⁻² has been found to be particularly advantageous.

MV is suitably measured using capillary rheometry operating at 400°C at a shear rate of 1000s⁻¹ using a tungsten carbide die, 0.5 mm x 3.175 mm.

The PAEK, e.g. PEEK, may also include other repeating units to form a copolymer. A particularly preferred copolymer contains repeating units of PEEK and polyether diphenyl ether ketone (PEDEK). Suitable copolymers of PEEK and PEDEK are disclosed in EP0184458A.

PEDEK is a polymer having a repeating unit of:

-O-Ph-Ph-O-Ph-CO-Ph (formula 2)

The PEEK-PEDEK copolymer is disclosed as having similar chemical resistance and mechanical properties to PEEK, but also as having a lower Tm than PEEK, but a similar or higher Tg value than PEEK.

WO 2014/207458 A1 discloses a suitable PEEK-PEDEK copolymer manufactured by a process comprising polycondensing a mixture of at least one dihydroxybenzene compound and at least one dihydroxybiphenyl compound in the molar proportions 65:35 to 95:5 with at least one dihalobenzophenone in the presence of sodium carbonate and potassium carbonate, where the mole% of potassium carbonate, used in the synthesis of the PEEK-PEDEK copolymer by nucleophilic polycondensation, is at least 2.5, where the mole% of potassium carbonate is expressed as a percentage of the total number of moles of the hydroxy monomers used in the synthesis. The PEEK-PEDEK copolymers of WO 2014/207458 A1 have higher crystallinity of the resulting PEEK-PEDEK copolymer compared to that disclosed that for the copolymers of EP0184458A.

Suitable PEEK-PEDEK copolymers are also disclosed in WO 2019/186085 A1 which discloses their formation by a specific nucleophilic polycondensation process including polymerisation-stopping (for instance using lithium salt) and end-capping of the copolymer (to provide specific end units to the copolymer), in the presence of reduced quantities of aromatic sulfone solvent, to provide PEEK-PEDEK copolymers with reduced chain branching and reduced melt viscosity at low shear rates compared to prior art copolymers of comparable molecular mass.

A filler material may be provided in the polymer material. Preferably, the filler comprises a fibrous filler material or a particulate filler material. Fibrous filler materials suitably have a longest dimension of 300 µm or less. Suitable fibrous filler materials may be selected from inorganic fibrous materials, organic fibrous materials, such as aramid fibres, and carbon fibre. Suitable fibrous filler materials may be selected from glass fibre, carbon fibre, alumina fibre or mixtures thereof. Preferred fibrous fillers are glass fibre and carbon fibre.

In a preferred embodiment, the filler material is a particulate filler material. Suitable particulate (or non-fibrous) filler materials may be selected calcium sulphate, chromium oxide, glass fibre, iron oxide, magnesium carbonate, silicon dioxide (quartz), zinc oxide, barium sulphate and boron nitride. The filler may be a bioactive material.

The bioactive material may comprise an antimicrobial, and/or a phosphate and/or a sulfate. Suitably, the bioactive material comprises a phosphate. Suitably, the bioactive material comprises a material selected from the group consisting of apatites, calcium phosphates and calcium sulfates. Suitably, the bioactive material comprises an apatite, preferably hydroxyapatite (HA). The bioactive material may comprise calcium phosphate. The bioactive material may comprise tri calcium phosphate. The bioactive material may comprise alpha and/or beta tri calcium phosphate.

The polymer material may comprise at least 5 wt% of the filler, suitably at least 10 wt% for example, 30wt% thereof. Suitably the filler material provides from 5 to 40 wt% of the polymeric material, preferably between 10 to 30wt% of the total composition of the polymeric material. For example, 12wt%, or 20wt% of filler material.

Another aspect of the invention relates to a three-dimensional object made by additive manufacturing.

The disclosure is not limited to the embodiments described above. Herein, many variations are conceivable within the scope of the appended claims and the clauses below. For instance, features of respective embodiments can be combined.

### Clauses

Clause 1. A method for three dimensional (3D) printing an object, the method comprising the steps of:
   - providing a print job, the print job comprising a representation of the object,
   - dividing the representation of the object in one or more bodies;
   - for each body, define settings for printing the respective body, wherein settings for at least one body include filling the body with a porous infill structure;
   - slicing the representation of the object in a number of slices,
   - the step of slicing comprising, for the body to be filled with the porous infill structure:
      - defining for every slice an infill area that is to be filled with the porous infill structure;
      - defining a circumference of the infill area;
      - adapting the porous infill structure to the circumference; and
      - filling the infill area with the adapted infill structure.
Clause 2. The method of clause 1, wherein the step of adapting the porous infill structure to the circumference comprises one or more of:
   - changing a pattern at least in part of non-orthogonal repetition;
   - executing a non-linear coordinate transformation of the infill structure;
   - adding additional tracks in parts of the infill pattern with low density.
Clause 3. The method of clause 1, wherein the step of adapting the porous infill structure to the circumference comprises changing the infill pattern to at least in part follow a curvature of the circumference.
Clause 4. The method of one of the previous clauses, comprising the step of printing the sliced print job to provide the object.
Clause 5. The method of one of the previous clauses, wherein the porous infill structure comprises a pattern having a unit cell which is repeated in an orthogonal grid.
Clause 6. The method of clause 5, the step of adapting the porous infill structure to the circumference including rendering at least part of the infill structure non-orthogonal.
Clause 7. The method of one of the previous clauses, the step of adapting the porous infill structure to the circumference including changing a repetitive pattern of at least part of the infill structure.
Clause 8. The method of one of the previous clauses, the step of slicing comprising, for the body to be filled with the porous infill structure:
   - identify one or more points on the circumference of the infill area;
   - connecting the one or more points with one or more extended lines;
   - creating offsets to the one or more extended lines towards an inside of the respective slice;
   - trimming the offsets to the circumference of the infill area.
Clause 9. The method of one of the previous clauses, the step of slicing comprising, for the part to be filled with the porous infill structure:
   - filling the infill area with the porous infill structure;
   - for a percentage of the slices of the respective part, adding tracks following the circumference of the infill area; and
   - removing tracks of the infill structure which are within a threshold distance with respect to the circumference of the infill area.
Clause 10. The method of clause 9, the step of slicing the part to be filled with the infill structure comprising:
   - identify one or more points on the circumference of the infill area;
   - connecting the one or more points with one or more lines, said lines defining the circumference.
Clause 11. The method of clause 9 or 10, wherein the percentage is in the range of 25 to 75%, for instance about 50%.
Clause 12. The method of one of clauses 1 to 7, the step of slicing comprising, for the part to be filled with the infill structure:
   - adapting a grid to the circumference of the infill area to create a virtual grid;
   - projecting the virtual grid on the infill area;
   - projecting the infill structure on the virtual grid.
Clause 13. The method of clause 12, the step of adapting a grid to the circumference of the infill area to create a virtual grid comprising changing a distance between tracks of the grid within a range.
Clause 14. The method of clause 12 or 13, the step of adapting a grid to the circumference of the infill area to create a virtual grid comprising changing a distance between adjacent ends of tracks of the grid within a range.
Clause 15. The method of one of clauses 12 to 14, the step of adapting a grid to the circumference of the infill area to create a virtual grid comprising changing a number of tracks of the grid within a range to optimize an average distance between said tracks.
Clause 16. A 3D printing system adapted to execute a method according to one of clauses 1 to 15.
Clause 17. A three-dimensional object made using the method of one of clauses 1 to 15.
Clause 18. A three-dimensional printed object, comprising:
   at least two bodies, wherein at least one body has a porous structure extending to an outer surface of the object;
   wherein a majority of ends of first tracks of the porous structure facing the outer surface are supported by second tracks.
Clause 19. The object of clause 18, wherein an unsupported length m of the ends of the first tracks facing the outer surface is at most 1.99, 1.8, 1.69, 1.5, 1.4, 1.39, 1.2, 1.19, 1, 0.99, 0.9, 0.84, 0.8, 0.7, 0.69, 0.65, 0.6, 0.59, 0.55, 0.5, 0.49 times a track width of said first tracks.
Clause 20. The object of clause 18 or 19, wherein the ends of the first tracks facing the outer surface have a radius R1 exceeding 0.35 times the track width of the first tracks.
Clause 21. The object of clause 20, wherein the radius R1 is in the order of 0.4, 0.42, 0.44, 0.45, 0.46, 0.50, 0.53, 0.55, 0.57, 0.6., 0.64, 0.65, 0.68, 0.7 times the track width of the first tracks or more.
Clause 22. The object of clause 20, wherein the radius R1 is in the range of about 0.3 to 0.85 times the track width of the first tracks.
Clause 23. The object of one of clauses 18 to 22, wherein at least 50%, 51%, 55%, 60%, 61%, 63%, 65%, 70%, 71%, 75%, 80%, 81%, 91% of the ends of the first tracks of the porous structure facing the outer surface are supported by second tracks.
Clause 24. The object of one of clauses 18 to 23, wherein spacings (pₕ) in between respective tracks provide openings to the internal volume of the object, thereby providing porosity, wherein the spacings are at least 0,1 times the width of the tracks.
Clause 25. The object of clause 24, wherein the openings are at least 0.2, for instance about 0.3 times the width of the tracks.
Clause 26. The object of clause 24 or 25, wherein the porosity exceeds 40%.
Clause 27. The object of clause 26, wherein the porosity exceeds 43%, for instance about 45%, 48%, 50%, 55%, 60%, 65%, 70%, or 75%.
Clause 28. The object of one of clauses 18 to 27, wherein a track width is about 1.2 to 10 times a layer height.
Clause 29. The object of one of clauses 18 to 28, wherein the object is a medical implantable device or a filter.
Clause 30. The object of clause 29, wherein the medical implantable device is selected from:
   - Craniomaxillofacial implant;
   - Trauma plates;
   - Cervical implants;
   - Lumbar interbody fusion implants (spine, thorax region and lower back);
   - Hip cups;
   - Knee implants; and
   - Ankle plates.
Clause 31. The object of one of clauses 18 to 30, wherein the object is made of a material comprising a thermoplastic polymer composition selected from the group of polyethylene, polypropylene, ABS, polycarbonate, polyamide and polyarylether ketones (PAEK), polyether ketone (PEK), polyether ether ketone (PEEK), polyether ketone ketone (PEKK), polyether ether ketone ketone (PEEKK), polyether ether ketone ether ketone ketone (PEKEKK), and combinations thereof.
Clause 32. The object of clause 31, wherein the thermoplastic polymer composition comprises at least 80 wt% of a PAEK, for instance at least 90 wt% of a PAEK, or the thermoplastic polymer composition comprises at least 80 wt% of a PEEK, for instance at least 90 wt% of a PEEK, for instance 100 wt% PEEK.

## Claims

1. A method for three dimensional (3D) printing an object, the method comprising the steps of:
- providing a print job, the print job comprising a representation of the object,
- dividing the representation of the object in one or more bodies;
- for each body, define settings for printing the respective body, wherein settings for at least one body include filling the body with a porous infill structure;
- slicing the representation of the object in a number of slices,
- the step of slicing comprising, for the body to be filled with the porous infill structure:
- defining for every slice an infill area that is to be filled with the porous infill structure;
- defining a circumference of the infill area;
- adapting the porous infill structure to the circumference; and
- filling the infill area with the adapted infill structure.

2. The method of claim 1, wherein the step of adapting the porous infill structure to the circumference comprises one or more of:
- changing a pattern at least in part of non-orthogonal repetition;
- executing a non-linear coordinate transformation of the infill structure;
- adding additional tracks in parts of the infill pattern with low density,
or
wherein the step of adapting the porous infill structure to the circumference comprises changing the infill pattern to at least in part follow a curvature of the circumference.

3. The method of one of the previous claims, wherein the porous infill structure comprises a pattern having a unit cell which is repeated in an orthogonal grid, wherein optionally, the step of adapting the porous infill structure to the circumference including rendering at least part of the infill structure non-orthogonal.

4. The method of one of the previous claims, the step of adapting the porous infill structure to the circumference including changing a repetitive pattern of at least part of the infill structure.

5. The method of one of the previous claims,
the step of slicing comprising, for the body to be filled with the porous infill structure:
- identify one or more points on the circumference of the infill area;
- connecting the one or more points with one or more extended lines;
- creating offsets to the one or more extended lines towards an inside of the respective slice;
- trimming the offsets to the circumference of the infill area, and/or the step of slicing comprising, for the part to be filled with the porous infill structure:
- filling the infill area with the porous infill structure;
- for a percentage of the slices of the respective part, adding tracks following the circumference of the infill area; and
- removing tracks of the infill structure which are within a threshold distance with respect to the circumference of the infill area, and optionally wherein the step of slicing the part to be filled with the infill structure comprises:
- identify one or more points on the circumference of the infill area;
- connecting the one or more points with one or more lines, said lines defining the circumference.

6. The method of one of claims 1 to 4, the step of slicing comprising, for the part to be filled with the infill structure:
- adapting a grid to the circumference of the infill area to create a virtual grid;
- projecting the virtual grid on the infill area;
- projecting the infill structure on the virtual grid,
and wherein optionally:
- the step of adapting a grid to the circumference of the infill area to create a virtual grid comprises changing a distance between tracks of the grid within a range; and/or
- the step of adapting a grid to the circumference of the infill area to create a virtual grid comprising changing a distance between adjacent ends of tracks of the grid within a range; and/or
- the step of adapting a grid to the circumference of the infill area to create a virtual grid comprising changing a number of tracks of the grid within a range to optimize an average distance between said tracks.

7. A 3D printing system adapted to execute a method according to one of claims 1 to 6.

8. A three-dimensional object made using the method of one of claims 1 to 6.

9. A three-dimensional printed object, comprising:
at least two bodies, wherein at least one body has a porous structure extending to an outer surface of the object;
wherein a majority of ends of first tracks of the porous structure facing the outer surface are supported by second tracks.

10. The object of claim 9, wherein an unsupported length m of the ends of the first tracks facing the outer surface is at most 1.99, 1.8, 1.69, 1.5, 1.4, 1.39, 1.2, 1.19, 1, 0.99, 0.9, 0.84, 0.8, 0.7, 0.69, 0.65, 0.6, 0.59, 0.55, 0.5, 0.49 times a track width of said first tracks.

11. The object of claim 9 or 10, wherein the ends of the first tracks facing the outer surface have a radius R1 exceeding 0.35 times the track width of the first tracks, wherein optionally the radius R1 is in the order of 0.4, 0.42, 0.44, 0.45, 0.46, 0.50, 0.53, 0.55, 0.57, 0.6., 0.64, 0.65, 0.68, 0.7 times the track width of the first tracks or more and/or wherein the radius R1 is in the range of about 0.3 to 0.85 times the track width of the first tracks.

12. The object of one of claims 9 to 11, wherein at least 50%, 51%, 55%, 60%, 61%, 63%, 65%, 70%, 71%, 75%, 80%, 81%, 91% of the ends of the first tracks of the porous structure facing the outer surface are supported by second tracks.

13. The object of one of claims 9 to 12, wherein spacings (pₕ) in between respective tracks provide openings to the internal volume of the object, thereby providing porosity, wherein the spacings are at least 0,1 times the width of the tracks, wherein optionally:
- the openings are at least 0.2, for instance about 0.3 times the width of the tracks, and/or
- the porosity exceeds 40%, or exceeds 43%, for instance about 45%, 48%, 50%, 55%, 60%, 65%, 70%, or 75%.

14. The object of one of claims 9 to 13, wherein a track width is about 1.2 to 10 times a layer height.

15. The object of one of claims 9 to 14, wherein:
- the object is a medical implantable device or a filter, wherein optionally the medical implantable device is selected from:
• Craniomaxillofacial implant;
• Trauma plates;
• Cervical implants;
• Lumbar interbody fusion implants (spine, thorax region and lower back);
• Hip cups;
• Knee implants; and
• Ankle plates; and/or
- the object is made of a material comprising a thermoplastic polymer composition selected from the group of polyethylene, polypropylene, ABS, polycarbonate, polyamide and polyarylether ketones (PAEK), polyether ketone (PEK), polyether ether ketone (PEEK), polyether ketone ketone (PEKK), polyether ether ketone ketone (PEEKK), polyether ether ketone ether ketone ketone (PEKEKK), and combinations thereof.
